(19) Europäisches Patentamt / European Patent Office / Office européen des brevets

(11) EP 4 168 778 B1

(12) **EUROPÄISCHE PATENTSCHRIFT**

(45) Veröffentlichungstag und Bekanntmachung des Hinweises auf die Patenterteilung:
**31.07.2024 Patentblatt 2024/31**

(21) Anmeldenummer: **21735914.0**

(22) Anmeldetag: **23.06.2021**

(51) Internationale Patentklassifikation (IPC):
***G01N 21/64*** *(2006.01)* ***G02B 21/00*** *(2006.01)*

(52) Gemeinsame Patentklassifikation (CPC):
**G01N 21/6458; G02B 21/0076;** G02B 21/16

(86) Internationale Anmeldenummer:
**PCT/EP2021/067068**

(87) Internationale Veröffentlichungsnummer:
**WO 2021/259967 (30.12.2021 Gazette 2021/52)**

(54) **VERFAHREN ZUR LOKALISATION EINZELNER MOLEKÜLE EINES FARBSTOFFS IN EINER PROBE UND ZUM ERZEUGEN HOCHAUFGELÖSTER BILDER EINER STRUKTUR IN EINER PROBE**

METHOD FOR LOCATING INDIVIDUAL MOLECULES OF A DYE IN A SPECIMEN AND FOR GENERATING HIGH-RESOLUTION IMAGES OF A STRUCTURE IN A SPECIMEN

PROCÉDÉ POUR LOCALISER DES MOLÉCULES INDIVIDUELLES D'UN COLORANT DANS UN ÉCHANTILLON ET POUR PRODUIRE DES IMAGES HAUTE RÉSOLUTION D'UNE STRUCTURE DANS UN ÉCHANTILLON

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.06.2020 DE 102020116547**

(43) Veröffentlichungstag der Anmeldung:
**26.04.2023 Patentblatt 2023/17**

(73) Patentinhaber: **Abberior Instruments GmbH**
**37077 Göttingen (DE)**

(72) Erfinder: **KASTRUP, Lars**
**37037 Göttingen (DE)**

(56) Entgegenhaltungen:
- **GWOSCH KLAUS C ET AL: "MINFLUX nanoscopy delivers 3D multicolor nanometer resolution in cells", NATURE METHODS, NATURE PUB. GROUP, NEW YORK, vol. 17, no. 2, 13 January 2020 (2020-01-13), pages 217 - 224, XP037006746, ISSN: 1548-7091, [retrieved on 20200113], DOI: 10.1038/S41592-019-0688-0**
- **PETR KLÁN ET AL: "Photoremovable Protecting Groups in Chemistry and Biology: Reaction Mechanisms and Efficacy", CHEMICAL REVIEWS, vol. 113, no. 1, 9 January 2013 (2013-01-09), pages 119 - 191, XP055049223, ISSN: 0009-2665, DOI: 10.1021/cr300177k**
- **HESS S T ET AL: "Ultra-high resolution imaging by fluorescence photoactivation localization microscopy", BIOPHYSICAL JOURNAL, ELSEVIER, AMSTERDAM, NL, vol. 91, no. 11, 1 December 2006 (2006-12-01), pages 4258 - 4272, XP008082813, ISSN: 0006-3495, DOI: 10.1529/BIOPHYSJ.106.091116**
- **FRANCISCO BALZAROTTI ET AL: "Nanometer resolution imaging and tracking of fluorescent molecules with minimal photon fluxes", SCIENCE, vol. 355, no. 6325, 10 February 2017 (2017-02-10), US, pages 606 - 612, XP055426923, ISSN: 0036-8075, DOI: 10.1126/science.aak9913**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 4 168 778 B1

## Beschreibung

### Technisches Gebiet der Erfindung

Die Erfindung betrifft Verfahren zum Erzeugen hochaufgelöster Bilder einer Struktur bzw. zur Lokalisation einzelner Moleküle eines Fluoreszenzfarbstoffs in einer Probe. Hierzu wird die Probe oder ein Ausschnitt der Probe mit Anregungslicht und einer ein lokales Minimum aufweisenden Intensitätsverteilung von Fluoreszenzverhinderungslicht abgetastet, das die Emission von Fluoreszenzlicht durch den Fluoreszenzfarbstoff verhindert, reduziert oder gänzlich unterdrückt. Gegenüber verwandten, aus dem Stand der Technik bekannten Verfahren zeichnet sich das erfindungsgemäße Verfahren dadurch aus, dass der Fluorophor vor der Abtastung mit Anregungs- und Fluoreszenzverhinderungslicht in einer mindestens zwei Reaktionsschritte umfassenden Fotoaktivierungsreaktion zunächst aus einer geschützten, nicht fluoreszenten Form des Farbstoffs gebildet wird und dass die geschützte, nicht-fluoreszente Form des Farbstoffs inert ist gegenüber dem Anregungs- und Fluoreszenzverhinderungslicht.

### Stand der Technik

Unter STED-Mikroskopie wird ein Verfahren der Laserscanningmikroskopie verstanden, das eine räumlich hochaufgelöste Abbildung einer mit einem Fluoreszenzfarbstoff markierten Probe ermöglicht. Dabei wird die Probe mit fokussiertem Anregungslicht und mit Stimulationslicht abgetastet, wobei die Intensitätsverteilungen des Anregungs- und des Stimulationslichts weitgehend komplementär sind und die Intensitätsverteilung des Stimulationslichts am Ort des Intensitätsmaximums des Anregungslichts eine Nullstelle aufweist. An Orten hoher Intensität hindert das Stimulationslicht den Fluoreszenzfarbstoff an der Emission von Fluoreszenz, wodurch die Fluoreszenzemission auf einen kleinen Bereich um die Nullstelle der Intensitätsverteilung des Stimulationslichts eingeschränkt und die Größe der effektiv wirksamen Punktspreizfunktion reduziert wird.

Als eine Weiterbildung der STED-Mikroskopie ist in der DE 10 2013 100 172 A1 ein Verfahren zur STED-Mikroskopie offenbart, bei dem die Probe im Messbereich vor dem Beleuchten mit Anregungslicht und Stimulationslicht zusätzlich mit einer - ebenso wie das Stimulationslicht - ein lokales Minimum aufweisenden Intensitätsverteilung von Fluoreszenzverhinderungslicht beleuchtet wird, das den Fluorophor aus dem fluoreszenten Zustand in einen Schutzzustand überführt, in dem der Fluorophor vor elektronischer Anregung durch das Anregungslicht und das Stimulationslicht geschützt ist. Dabei besteht das Ziel des Verfahrens nicht vorrangig darin, die Auflösungsvermögen des Mikroskops durch Überlagern der Intensitätsverteilungen des Stimulations- und des Fluoreszenzverhinderungslichts zu verbessern; vielmehr soll das Fotobleichen des Fluorophors durch das Anregungs- und das Stimulationslicht insbesondere in Bereichen hoher Intensität des Stimulationslichts reduziert werden, indem der Fluorophor besonders in diesen Bereichen in den nicht anregbaren Schutzzustand überführt und somit vor der bleichenden Wirkung des Anregungs- und Stimulationslichts geschützt wird. Voraussetzung für die Anwendung des Verfahrens ist die Verfügbarkeit geeigneter Fluorophore, die temporär in den Schutzzustand überführt werden können, d.h. fotoschaltbar sind. Dabei sind die Anforderungen an den Schaltkontrast weniger streng als beispielsweise bei der RESOLFT-Mikroskopie, allerdings muss der Schutzzustand (weitgehend) inert gegenüber dem Anregungs-und dem Stimulationslicht sein - eine Anforderung, die bei vielen fotoschaltbaren Fluorophoren in der Praxis nicht oder nur unzureichend erfüllt ist.

Unter dem Sammelbegriff der Lokalisationsmikroskopie sind weitere, miteinander verwandte Verfahren zur hochauflösenden Fluoreszenzmikroskopie bekannt. Diese Methoden beruhen auf der hochgenauen räumlichen Lokalisation einzelner fluoreszierender Farbstoffmoleküle und der Rekonstruktion eines hochaufgelösten Bildes aus diesen Einzelmolekül-Lokalisationen. Voraussetzung für die Anwendung dieser Methoden ist, dass in der Probe zu jedem Zeitpunkt fluoreszierende Farbstoffmoleküle vereinzelt, d.h. räumlich voneinander getrennt vorliegen, so dass sie bei einer Abbildung auf eine Kamera als isolierte Objekte im Kamerabild erscheinen. Räumlich vereinzelt vorliegende Fluoreszenzfarbstoffmoleküle können zwar beispielsweise durch Verwendung einer entsprechend hohen Verdünnung des Farbstoffs bei der Markierung der Probe erreicht werden; um eine hochaufgelöste, räumlich möglichst kontinuierliche Abbildung der Struktur generieren zu können, ist es aber erforderlich, dass die Struktur mit vielen, in großer Dichte angeordneten Fluoreszenzfarbstoffmolekülen markiert ist und dass die Orte eines hinreichend großen Teils dieser Fluoreszenzfarbstoffmoleküle, typischerweise mehrerer Tausend Fluoreszenzfarbstoffmoleküle, bestimmt werden. Die notwendigerweise hohe Markierungsdichte mit Fluoreszenzfarbstoffmolekülen bedingt, dass während einer Lokalisation von Farbstoffmolekülen immer nur ein kleiner Anteil aller Farbstoffmoleküle in einem fluoreszenten Zustand vorliegen darf, um die Anforderung, dass fluoreszente Moleküle einzeln und räumlich isoliert vorliegen, erfüllen zu können. In der Lokalisationsmikroskopie greift man daher auf fotoschaltbare Fluoreszenzfarbstoffe zurück, die einen fluoreszenten Zustand aufweisen, in dem der Farbstoff mit Anregungslicht geeigneter Wellenlänge zur Fluoreszenz angeregt werden kann, und weiterhin einen Dunkelzustand aufweisen, in dem der Farbstoff mit dem Anregungslicht nicht zur Fluoreszenz angeregt werden kann. Dabei lässt sich der Farbstoff zumindest einmal fotoaktivieren, d.h. aus dem Dunkelzustand in den fluoreszenten Zustand überführen. Die Fotoaktivierung erfolgt oft lichtinduziert, d. h. durch Beleuchtung mit Fotoaktivierungslicht geeigneter Wellenlänge (meist im blau-violetten Spektralbereich), wodurch der Anteil fotoaktivierter Farbstoffmoleküle genau eingestellt und kontrolliert werden kann. Alternativ können Farbstoffmoleküle auch spontan in einen aktivierten Zustand wechseln. Je nach Art des Fluoreszenzfarbstoffs kann die Aktivierung auch reversibel sein, d. h. es können mehrere Aktivierungs-Deaktivierungs-Schaltzyklen durchlaufen werden, wobei auch die Deaktivierung bzw. der Übergang in einen nicht-fluoreszenten Zustand lichtinduziert oder spontan erfolgen kann. Ein typischer Schaltmechanismus ist der (reversible) Übergang aus dem fluoreszenten Zustand in einen transienten Dunkelzustand, beispielsweise einen Triplettzustand. Die Schaltkinetiken dieser Übergänge können unter anderem über die Lösungsmittelzusammensetzung, durch Zusatz von Redox-Reagenzien und/oder durch Kontrolle der Sauerstoffkonzentration in der Probe an die jeweiligen Erfordernisse angepasst werden.

Die in der Lokalisationsmikroskopie erreichbare Lokalisationsgenauigkeit hängt von mehreren Faktoren ab und wurde beispielsweise von R.E. Thompson et al., Biophys. J. 82, 2775 (2002) wie folgt angegeben:

$$\sigma = \sqrt{\frac{s^2}{N} + \frac{a^2}{12N} + \frac{4\sqrt{\pi}s^3b^2}{aN^2}}, \qquad (1)$$

wobei *s* die Breite (Standardabweichung) einer als Gaußfunktion approximierten PSF des Mikroskops ist, *N* die Anzahl detektierter Fluoreszenzphotonen des Farbstoffmoleküls, *a* die Kantenlänge der Detektor-Pixel und *b* das sich aus Hintergrund-Fluoreszenz und Detektor-Rauschen zusammensetzende Hintergrundsignal.

Sofern die Lokalisationsgenauigkeit nicht wesentlich durch die Größe der Detektorpixel *a* oder durch das Hintergrundsignal *b* begrenzt ist, zeigt Gleichung (1), dass mit einer (effektiven) PSF geringerer Breite s bei einer gegebenen Photonenzahl *N* eine höhere Lokalisationsgenauigkeit bzw. eine kleinere Lokalisationsunsicherheit $\sigma$ erzielt wird bzw. eine angestrebte Lokalisationsgenauigkeit mit einer geringeren Photonenzahl *N* erreicht werden kann. Eine schmalere effektive PSF (mit einem kleineren Wert *s*) kann beispielsweise dadurch erzeugt werden, dass die Probe nicht flächig mit homogen verteiltem Anregungslicht beleuchtet wird, sondern in Analogie zur STED-Mikroskopie mit fokussiertem Anregungs- und Stimulationslicht, das mittels einer Abtastvorrichtung das Bildfeld abtastet. Dabei wird einem gaußförmigen Anregungsfokus eine ein lokales Intensitätsminimum aufweisende Intensitätsverteilung von Stimulationslicht überlagert, das eine Fluoreszenzemission in den Randbereichen des Anregungsfokus' unterdrückt und somit die Breite s der effektiven PSF reduziert. Da die abtastende Beleuchtung des gesamten Bildfeldes naturgemäß langsamer ist als eine flächige Weitfeldbeleuchtung, muss das Aufnahmeschema unter Umständen angepasst werden dahingehend, dass das Bildfeld nicht als Ganzes, sondern ausschnittsweise abgetastet wird, wobei die Ausschnitte beispielsweise so gewählt werden, dass sie jeweils ein fluoreszierendes Farbstoffmolekül enthalten.

Die neuere, erstmals von F. Balzarotti et al. in arXiv:1611.03401 [physics.optics] beschriebene MINFLUX-Nanoskopie verbindet Merkmale der genannten Verfahren. Dabei werden räumlich isolierte, fluoreszente Moleküle mit einer ein Intensitätsminimum aufweisenden Intensitätsverteilung von Anregungslicht an einer Abfolge von verschiedenen Positionen beleuchtet. Für jede der Beleuchtungspositionen wird die durch das Anregungslicht angeregte Fluoreszenzemission registriert, und es wird aus der Menge der registrierten Werte der Intensität des Fluoreszenzlichts auf die Position des fluoreszenten Moleküls geschlossen. Naturgemäß ist diese Positionsbestimmung mit einer Unsicherheit behaftet, wobei die Unsicherheit der Positionsbestimmung jedoch dadurch verringert werden kann, dass das Verfahren iterativ angewendet wird. Hierzu werden vor jedem Iterationsschritt die Beleuchtungspositionen angepasst, d. h. näher um die jeweils angenommene Position des Moleküls angeordnet. Gleichzeitig wird die Stärke des Anregungslichts erhöht, so dass der Intensitätsgradient in der Nähe des Intensitätsminimums zunimmt. Alternativ kann die Messdauer erhöht werden, was bezüglich der Menge des wirksamen Lichts einer Erhöhung der Stärke des Anregungslichts entspricht. Mit den angepassten Parametern wird das Molekül nacheinander an jeder der angepassten Beleuchtungspositionen beleuchtet und die Intensität der Fluoreszenzemission registriert. Aus der Abhängigkeit des Fluoreszenzsignals von den Positionen des Intensitätsminimums kann nun die Position des Moleküls mit geringerer Unsicherheit als zuvor bestimmt werden. Diese Verfahrensschritte können bis zur Konvergenz der Positionsbestimmung oder bis zum Erreichen eines anderen Abbruchkriteriums, beispielsweise einer vorher festgelegten maximal akzeptablen Unsicherheit, wiederholt werden. Mit einer erreichbaren Lokalisationsgenauigkeit von ca. 1 nm stellt das MI NFLUX-Verfahren nach dem aktuellen Stand der Technik die präziseste kommerziell erhältliche Lokalisationsmethode für fluoreszierende Moleküle dar.

Die Druckschrift WO 2015/097000 A1 offenbart weiter, dass aus den Positionsdaten der einzelnen Moleküle ein (hochaufgelöstes) Abbild der Verteilung der Moleküle in der Probe gewonnen werden kann ("MINFLUX Imaging"). Dieses Verfahren entspricht den aus der Lokalisationsmikroskopie allgemein bekannten Vorgehen zur Erzeugung hochaufgelöster Bilder aus einer Vielzahl von Positionsbestimmungen einzelner fluoreszenter Moleküle, resultiert im Falle der MINFLUX-Nanoskopie allerdings in einer nochmals gesteigerten räumlichen Auflösung der Bilder von 5 nm oder besser.

In der DE 10 2017 104 736 B3 ist eine Modifikation des MINFLUX-Verfahrens beschrieben, bei der das Abtasten der vereinzelt vorliegenden Fluoreszenzfarbstoffmoleküle nicht durch Beleuchten mit einer ein lokales Intensitätsminimum aufweisenden Intensitätsverteilung von Anregungslicht, sondern mit zwei im Wesentlichen komplementären Intensitätsverteilungen von Anregungs- und Fluoreszenzverhinderungslicht erfolgt. Dabei weist die Intensitätsverteilung des Anregungslichts ein lokales Intensitätsmaximum auf, während die Intensitätsverteilung des Fluoreszenzverhinderungslichts an derselben Stelle ein lokales Intensitätsminimum aufweist. Bei dem Fluoreszenzverhinderungslicht kann es sich konkret um Stimulationslicht handeln, das in den Randbereichen der Intensitätsverteilung des Anregungslichts angeregte Fluoreszenzfarbstoffmoleküle durch Auslösen stimulierter Emission an der Aussendung von Fluoreszenzphotonen hindert. Es werden also das Anregungslicht und das Fluoreszenzverhinderungslicht mit solchen Intensitätsverteilungen überlagert, wie dies auch bei der RESOLFT- und der STED-Mikroskopie geschieht. Es wird ausgenutzt, dass die Intensität des Fluoreszenzlichts, die für das jeweilige Fluoreszenzfarbstoffmolekül registriert wird, von dessen Abstand zu dem lokalen Intensitätsminimum des Fluoreszenzverhinderungslichts abhängt, und dass dessen Position mit hoher Genauigkeit aus den für mehrere Positionen des Intensitätsminimums des Fluoreszenzverhinderungslichts registrierten Intensitäten des Fluoreszenzlichts bestimmt werden kann. Auch bei dieser Modifikation des MINFLUX-Verfahrens kann das lokale Intensitätsminimum an wenigen Positionen in der Probe positioniert werden, und die Auswertung der Intensitäten des registrierten Fluoreszenzlichts kann nach denselben Prinzipien erfolgen wie bei der MINFLUX-Nanoskopie. Als Unterschied verbleibt jedoch, dass bei der MINFLUX-Nanoskopie die Intensität des Fluoreszenzlichts von dem Fluoreszenzmarker mit zunehmendem Abstand seiner Position zu der Position des lokalen Intensitätsminimums zunimmt, während sie bei der Modifikation des Verfahrens, bei dem das weitere Licht Fluoreszenzverhinderungslicht ist, mit zunehmendem Abstand abnimmt.

Die Publikation "Ultra-High Resolution Imaging by Fluorescence Photoactivation Localization Microscopy" von S.T. Hess, T.P.K. Girirajan und M.D. Mason (Biophysical Journal 91, 4258-4272 (2006)) beschreibt ein lokalisationsmikroskopisches Verfahren (FPALM), bei dem fotoaktivierbare Fluorophore mit einem hochfrequenten Aktivierungslaser in einen aktiven Zustand überführt werden und deren Beugungsbilder auf eine Kamera abgebildet werden, um die Position der Fluorophore mit hoher Präzision zu bestimmen. Dabei wird die Aktivierungslichtaktivität so eingestellt, dass die aktiven Fluorophore zu jedem Zeitpunkt einen Abstand oberhalb der Beugungsgrenze aufweisen. Dabei wird unter anderem eine Publikation zitiert, die eine ZweiPhotonen-Aktivierung von fotoaktivierbarem grünfluoreszierendem Protein beschreibt (Y. Chen, P.J. MacDonald, J.P. Skinner, G.H. Patterson, J.D. Muller: "Probing nucleocytoplasmic transport by two-photon activation of PA-GFP", Microsc.Res.Tech. 69;220-226 (2006)).

Zusammenfassend sind aus dem Stand der Technik mehrere Varianten hochauflösender Fluoreszenzmikroskopie bekannt, bei denen für das Erreichen hoher räumlicher Auflösung in der Abbildung neben Anregungslicht auch Stimulationslicht genutzt wird. Gleichzeitig erfordern diese Verfahren den Einsatz fotoaktivierbarer oder fotoschaltbarer Fluoreszenzfarbstoffe zur Markierung der Probe. In der Praxis ist die Anwendbarkeit dieser Verfahren eingeschränkt, da die üblicherweise genutzten fotoaktivierbaren Fluoreszenzfarbstoffe mit einer fotolabilen Schutzgruppe nicht oder nicht ausreichend inert sind insbesondere gegenüber der Beleuchtung mit Stimulationslicht hoher Intensität. Dies trifft in besonderem Maße zu, wenn das Stimulationslicht in Form kurzer Laserpulse mit sehr hohen Spitzenintensitäten - wie in der STED-Mikroskopie bevorzugt - eingesetzt wird. Dann kommt es verstärkt auch zu einer Mehrphotonenabsorption durch die fotolabilen Schutzgruppen, in deren Folge der Fluoreszenzfarbstoff in unerwünschter Weise fotoaktiviert wird.

## Aufgabe der Erfindung

Der Erfindung liegt daher die Aufgabe zu Grunde, Verfahren zur hochauflösenden Fluoreszenzmikroskopie anzugeben, bei denen fotoaktivierbare Fluoreszenzfarbstoffe hohen Intensitäten von Stimulationslicht oder anderem Fluoreszenzverhinderungslicht ausgesetzt werden können, ohne dass die fotoaktivierbaren Fluoreszenzfarbstoffe in verfahrensbehindernder Weise durch das Stimulationslicht bzw. Fluoreszenzverhinderungslicht aktiviert werden, sondern sich diesem gegenüber inert verhalten.

## Lösung

Die Aufgabe der Erfindung wird durch Verfahren nach den unabhängigen Ansprüchen 1, 6 und 9 gelöst. Die abhängigen Ansprüche 2 bis 5, 7 bis 8 und 10 bis 14 betreffen bevorzugte Ausführungsformen des Verfahrens. Die Verwendungsanspruch 15 richtet sich auf die Verwendung eines fotoaktivierbaren Fluoreszenzfarbstoffs in einem erfindungsgemäßen Verfahren.

Die erfindungsgemäßen Verfahren basieren auf den aus dem Stand der Technik bekannten Verfahren hochauflösender Fluoreszenzmikroskopie, bei denen für das Erreichen einer hohen räumlichen Auflösung in der Abbildung neben Anregungslicht auch Fluoreszenzverhinderungs- bzw. Stimulationslicht genutzt wird. Sie verbessern die bekannten Verfahren jedoch in entscheidender Weise dahingehend, dass eine Art der Fotoaktivierung von Fluoreszenzfarbstoffen verwendet wird, die eine unbeabsichtigte und störende Fotoaktivierung durch Stimulations- oder Fluoreszenzverhinderungslicht stark reduzieren oder gänzlich unterdrücken kann. Hierdurch wird der Anwendungsbereich gegenüber den bekannten Verfahren stark erweitert oder eine Anwendung des Verfahrens überhaupt erst ermöglicht.

Die Erfindung basiert auf der Erkenntnis der Anmelderin, dass die geschützten, nicht-fluoreszenten Fluoreszenzfarbstoffe üblicherweise zwar einen kleinen Absorptionsquerschnitt bei der Wellenlänge des Stimulationslichts aufweisen, dass dieser geringe Absorptionsquerschnitt aber angesichts der teils sehr hohen Intensitäten des Fluoreszenzverhinderungs- bzw. Stimulationslichts immer noch ausreichend ist, um zu einer unerwünschten Fotoaktivierung zu führen. Außerdem kann es unter Umständen zu einer Zwei- oder Mehrphotonenabsorption des Fluoreszenzverhinderungs- bzw. Stimulationslichts kommen, die ebenfalls zu einer Fotoaktivierung führen kann. Einige der zur Herstellung fotoaktivierbarer Fluoreszenzfarbstoffe genutzten fotolabilen Schutzgruppen sind sogar für die Mehrphotonenabsorption optimiert.

Die Stabilität der fotoaktivierbaren Fluoreszenzfarbstoffe gegenüber dem Fluoreszenzverhinderungs- bzw. Stimulationslicht kann nun dadurch signifikant verbessert werden, dass die Fotoaktivierung des geschützten, nicht fluoreszenten Farbstoffstoffs so gestaltet wird, dass die Aktivierung nicht in einem, sondern in mehreren Reaktionsschritten erfolgt. Wenn dabei jeder einzelne der Reaktionsschritte lichtinduziert ist und die Fluoreszenzfähigkeit erst nach dem letzten Reaktionsschritt hergestellt wird, erfordert die Fotoaktivierung daher die Absorption zweier (oder mehrerer) Photonen, wodurch sich eine der Zwei-/ Mehrphotonen-Fluoreszenz analoge, nichtlineare (d.h. quadratische, kubische, ...) Abhängigkeit der Fotoaktivierungsrate von der Lichtintensität ergibt. Wie aus der Zweiphotonen-Fluoreszenzanregung bekannt, ist das Phänomen der Zweiphotonenabsorption in der Praxis nur bei der Verwendung sehr kurzer Lichtpulse bedeutsam, was sich auf die Fotoaktivierung beim Übergang von der Einphotonenzur Zweiphotonenabsorption übertragen lässt. Die unerwünschte Fotoaktivierung kann so zurückgedrängt werden, was übrigens auch in Fall von Mehrphotonenabsorptionen gilt, die zu Absorptionsprozessen (noch) höherer Ordnung werden.

Zwar reduziert sich auch die Wahrscheinlichkeit für die Fotoaktivierung mit dediziertem Aktivierungslicht, dieses steht jedoch üblicherweise in ausreichender Intensität zur Verfügung und kann insbesondere unabhängig vom Stimulationslicht dosiert werden. Aufgrund der höheren Ordnung des Fotoaktivierungsprozesses bleibt die Fotoaktivierung auf ein kleineres Volumen beschränkt und erlaubt daher sogar eine räumlich präzisere Steuerung der Fotoaktivierung.

**Beschreibung der Erfindung**

Bei der Erfindung handelt es sich um drei durch ein- und dasselbe erfinderische Konzept verbundene Verfahren zum Erzeugen hochauflösender Bilder einer Struktur in einer Probe bzw. zur Lokalisation einzelner Moleküle eines Fluoreszenzfarbstoffs in einer Probe sowie die Verwendung eines Fluoreszenzfarbstoffs in einem derartigen Verfahren. Allen drei Verfahren ist gemeinsam, dass ein fotoaktivierbarer Fluoreszenzfarbstoff ausgewählt wird, bei dem das Fotoaktivieren mindestens zwei jeweils lichtinduzierte Reaktionsschritte umfasst. Weiter erfolgt erfindungsgemäß bei allen Verfahren ein Fotoaktivieren des Fluoreszenzfarbstoffs in Form einer mindestens zwei lichtinduzierte Reaktionsschritte umfassenden Aktivierungsreaktion, wobei der Fluorophor erst nach dem letzten der Reaktionsschritte ausgebildet wird und der Farbstoff seine fluoreszenten Eigenschaften erhält. Schließlich erfolgt in allen erfindungsgemäßen Verfahren ein Abtasten der Probe oder eines Ausschnitts der Probe mit einer ein Intensitätsminimum aufweisenden Intensitätsverteilung von Anregungs- und/oder Fluoreszenzverhinderungslicht.

Das erste erfindungsgemäße Verfahren umfasst die einleitenden Schritte

1. Auswahl eines Fluoreszenzfarbstoffs, der so beschaffen ist, dass er initial in einer geschützten, nicht-fluoreszenten Form vorliegt und der durch Beleuchten mit Aktivierungslicht aus der geschützten in eine aktivierte, fluoreszente Form überführbar, d.h. fotoaktivierbar ist. Unter fluoreszent wird hier verstanden, dass der Farbstoff mit Licht geeigneter Wellenlänge zur Abgabe von Fluoreszenzlicht angeregt werden kann, während dies in der nicht-fluoreszenten Form nicht möglich ist. Erfindungsgemäß wird der Fluoreszenzfarbstoff so ausgewählt, dass das Fotoaktivieren mindestens zwei jeweils lichtinduzierte Reaktionsschritte umfasst.

2. Markieren der interessierenden Struktur in der Probe mit dem ausgewählten Farbstoff, wozu aus dem Stand der Technik bekannte Methoden der Fluoreszenzmarkierung, unter anderem die Technik der Immunofluoreszenzmarkierung, herangezogen werden können.

Die weiteren Schritte des Verfahrens können ein- oder mehrmals ausgeführt werden und umfassen:
3. Fotoaktivieren einer (kleinen) Teilmenge des Fluoreszenzfarbstoffs aus der geschützten, nicht-fluoreszenten Form in die aktivierte, fluoreszente Form durch Beleuchten der Probe mit Aktivierungslicht. Die Teilmenge kann dabei räumlich definiert sein und beispielsweise Moleküle des Fluoreszenzfarbstoffs innerhalb eines Ausschnitts oder einer Ebene der Probe umfassen, die Teilmenge kann aber auch statistisch definiert sein und eine zufällige Auswahl aller Moleküle beinhalten. Insbesondere kann die Teilmenge einzelne, räumlich voneinander getrennte Moleküle umfassen.

Erfindungsgemäß erfolgt das Fotoaktivieren in Form einer mindestens zwei lichtinduzierte Reaktionsschritte umfassenden Aktivierungsreaktion, wobei der Fluorophor erst nach dem letzten der Reaktionsschritte ausgebildet wird und der Farbstoff seine fluoreszenten Eigenschaften erhält. Mit der Fotoaktivierung steigt die Fluoreszenzquantenausbeute des Farbstoffs, d. h. die Wahrscheinlichkeit, dass ein elektronisch angeregtes Farbstoffmolekül seine Anregungsenergie in Form eines Fluoreszenzphotons, und nicht durch nichtradiative Prozesse wieder abgibt, stark an. Da die Fluoreszenzquantenausbeute der geschützten Form in der Regel sehr klein, aber nicht exakt gleich null ist, ist der mit der Fotoaktivierung erzielte Fluoreszenzkontrast jedoch in der Praxis begrenzt. Als Fluoreszenzkontrast wird daher in der Regel mindestens ein Verhältnis von 1 : 10, bevorzugt von mindestens 1 : 100 und idealerweise von mindestens 1 : 1000 angestrebt.

Die Ausgestaltung des Fotoaktivierungsprozesses in zwei oder mehr Reaktionsschritten ist dabei durch unterschiedliche Reaktionsschemata realisierbar. Eine mögliche Ausgestaltung des Fotoaktivierungsprozesses besteht in der Anbindung mehrerer fotolabiler Schutzgruppen an funktionelle Gruppen des Fluoreszenzfarbstoffs dergestalt, dass sich erst nach der (parallelen oder sequenziellen) fotolytischen Abspaltung aller Schutzgruppen der Fluorophor ausbilden kann. Potenzielle Anbindungsstellen stellen insbesondere im Fluorophor enthaltene Hydroxy-, Amino-, Carbonyl- und Carboxylgruppen dar. Für diese funktionellen Gruppen sind zahlreiche fotolabile Schutzgruppen bekannt, beispielsweise o-Nitrobenzylgruppen, Benzoingruppen, Phenacylgruppen, Cumaryl- und Arylmethylgruppen, Benzoingruppen, Arylsulfonamide sowie substituierte Varianten dieser Gruppen. Für eine umfassende Übersicht sei auf den Review-Artikel "Photoremovable Protecting Groups in Chemistry and Biology: Reaction Mechanisms and Efficacy" von P. Klän et al. in Chem. Rev. 113, 119 (2013) verwiesen. Schutzgruppen der genannten Art sind auch zur Herstellung fotoaktivierbarer Fluoreszenzfarbstoffe geeignet, Beispiele geschützter Rhodamin- und Fluoreszein-Farbstoffe sind dem Fachmann aus dem Stand der Technik bekannt [siehe beispielsweise "Specific protein labeling with caged fluorophores for dual-color imaging and super-resolution microscopy in living cells", S. Hauke et al., Chem. Sci. 8, 559 (2017)].

Während die meisten fotolabilen Schutzgruppen mit UV-Licht abspaltbar sind, existiert auch eine zunehmende Zahl von Schutzgruppen, die im sichtbaren Spektralbereich abgespalten werden können. Hierzu zählen insbesondere Cumarylschutzgruppen, die mit blauem Licht (400 - 500 nm), und vom Bordipyrromethen (BODIPY) abgeleitete Schutzgruppen (siehe beispielsweise "BODIPY-Derived Photoremovable Protecting Groups Unmasked with Green Light", P. P. Goswami et al., J. Am. Chem. Soc. 137, 3783 (2015), die mit grünem Licht (> 500 nm) abspaltbar sind. Die inzwischen große spektrale Bandbreite der Schutzgruppen erlaubt auch eine selektive Abspaltung unterschiedlicher Schutzgruppen aus einem Molekül, siehe hierzu "Wavelength-Selective Cleavage of Photoprotecting Groups: Strategies and Applications in Dynamic Systems", M.J. Hansen et al., Chem. Soc. Rev. 44, 3358 (2015).

Die Abspaltung der Schutzgruppen kann mit monochromatischem Aktivierungslicht erfolgen. Insbesondere, wenn die fotolabilen Schutzgruppen chemisch identisch oder zumindest mit Licht gleicher Wellenlänge abspaltbar sind, lässt sich das Verfahren dann mit nur einer einzigen Aktivierungslichtquelle implementieren. Diese Aktivierungslichtquelle kann eine kontinuierliche oder auch eine gepulste Lichtquelle sein. Im letzteren Fall, insbesondere bei Verwendung von Ultrakurzpulslasern als Aktivierungslichtquelle, kann die Fotoaktivierung auch durch eine Zwei- oder Mehrphotonenabsorption initiiert werden. Alternativ kann das Aktivierungslicht aber auch mehrere Wellenlängen, insbesondere auch aus mehreren Lichtquellen, umfassen, wenn die Schutzgruppen verschieden sind und spektral unterschiedliche Absorptionscharakteristiken aufweisen.

Die große Variabilität der Schutzgruppen erlaubt ein auf den Einzelfall abgestimmtes Design des fotoaktivierbaren Fluoreszenzfarbstoffs, wobei eine Optimierung im Hinblick auf eine möglichst geringe Fotoaktivierung durch das Fluoreszenzverhinderungs- bzw. Stimulationslicht bei gleichzeitig möglichst großem Absorptionsquerschnitt für das Aktivierungslicht erfolgen kann. Diesbezüglich kann es vorteilhaft sein, dass sich die Schutzgruppen chemisch und in ihren spektralen Absorptionscharakteristika voneinander unterscheiden. In diesem Fall kann das Aktivierungslicht auch mehrere Wellenlängenkomponenten aufweisen, so dass auch chemisch unterschiedliche und in unterschiedlichen Spektralbereichen absorbierende Schutzgruppen abgespalten werden können. Die unterschiedlichen Wellenlängen des Aktivierungslichts können dabei gleichzeitig oder nacheinander eingestrahlt werden.

In den genannten Varianten kann die Abspaltung der mehreren Schutzgruppen dabei gleichzeitig bzw. zeitlich parallel erfolgen oder auch nacheinander. Eine streng sequenzielle Abfolge der zwei lichtinduzierten Reaktionsschritte erfolgt auch in einer anderen, vorteilhaften Variante des Verfahrens, bei der eine sogenannte Tandem-Reaktion genutzt wird (die nicht notwendigerweise in der Abspaltung einer Schutzgruppe resultieren muss). Unter Tandem-Reaktionen werden Reaktionsfolgen verstanden, die aus mehreren unabhängigen Reaktionsstufen (nicht aber mechanistischen Schritten) bestehen, die nacheinander ablaufen. Während unter Tandem-Reaktionen gemeinhin nur *spontan* nacheinander ablaufende Reaktionen verstanden werden, schließt der Begriff im Kontext dieses Dokuments explizit auch solche Reaktionen mit ein, deren Stufen einzeln und nacheinander - insbesondere durch Lichteinwirkung - initiiert werden. Erfindungsgemäß erfolgt die Herstellung des fluoreszenten Zustands erst nach der zweiten (oder einer weiteren) lichtinduzierten Stufe der Tandem-Reaktion. Ein Beispiel für eine derartige Tandem-Reaktion wurde von A. Paul et al. in "o-Hydroxycinnamate for Sequential Photouncaging of Two Different Functional Groups and its Application in Releasing Cosmeceuticals", Org. Biomol. Chem. 33 (2019) angegeben:

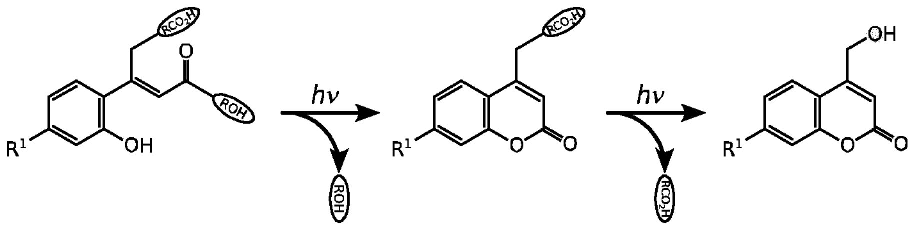


Einzelne Stufen der Tandem-Reaktion können auch reversibel sein, so dass der Ausgangszustand oder ein Zwischenzustand der Fotoaktivierung wiederhergestellt wird, wenn nachfolgende Reaktionsstufen nicht ausgelöst werden.

4. Ausbilden von Intensitätsverteilungen von Anregungslicht und Fluoreszenzverhinderungslicht in der Probe, wobei die Intensitätsverteilung des Fluoreszenzverhinderungslichts ein lokales Intensitätsminimum aufweist. Wie in der STED-Mikroskopie üblich wird die Intensitätsverteilung des Anregungslichts dabei bevorzugt in Form eines beugungsbegrenzt fokussierten Lichtflecks ausgebildet, dessen (zentrales) Intensitätsmaximum räumlich mit dem Intensitätsminimum des Fluoreszenzverhinderungslichts überlagert wird. Prinzipiell sind aber auch andere Intensitätsverteilungen des Anregungslichts denkbar und zur Durchführung des Verfahrens geeignet, beispielsweise eine homogene Verteilung von Anregungslicht in der Probe.

Unter dem Begriff des Fluoreszenzverhinderungslichts ist jede Art von Licht zu verstehen, die geeignet ist, die Fluoreszenz der aktivierten, fluoreszenten Farbstoffmoleküle bei der Beleuchtung mit Anregungslicht zu verhindern, zu reduzieren oder gänzlich zu unterdrücken. Insbesondere kann Fluoreszenzverhinderungslicht aus der STED-Mikroskopie bekanntes Stimulationslicht sein, das eine stimulierte Emission elektronisch angeregter Farbstoffmoleküle induziert, wodurch die Farbstoffmoleküle (zurück) in den elektronischen Grundzustand überführt und an einer spontanen Fluoreszenzemission gehindert werden. Wie aus der RESOLFT-Mikroskopie bekannt, kann Fluoreszenzverhinderungslicht auch lichtinduzierte chemische Reaktionen, insbesondere Isomerisierungs-, Zyklisierungs-/ Zykloreversionsreaktionen, auslösen, die mit einer Modulation der Fluoreszenzemission einhergehen.

Die Überlagerung des Anregungslichts mit dem Fluoreszenzverhinderungslicht führt zu einer Einengung des Bereichs, in dem mit Anregungslicht beleuchtete, fluoreszente Farbstoffmoleküle Fluoreszenzlicht abgeben können, und somit zu einer in ihrer Größe reduzierten effektiven PSF. Unter dem Auflösungsvermögen Δ wird hier gemäß der gängigen Praxis derjenige Abstand zwischen zwei identischen Punktobjekten verstanden, für den die zwei Punktobjekte noch als separate Objekte trenn- und abbildbar sind. Das bei der Abtastung erzielte Auflösungsvermögen hängt von der Intensität des Fluoreszenzverhinderungslichts ab und ist - wie dem Fachmann aus der STED- und der RESOLFT-Mikroskopie bekannt - gegeben durch:

$$\Delta = \frac{\lambda}{2n \sin\alpha \sqrt{1 + \frac{I}{I_s(\lambda)}}} \qquad (2)$$

wobei $\lambda$ die Wellenlänge des Fluoreszenzverhinderungslichts ist, n der Brechungsindex eines optischen Materials, in dem die Intensitätsverteilung des Fluoreszenzverhinderungslichts ausgebildet wird, $\alpha$ der halbe Öffnungswinkel einer optischen Anordnung, mit der die Probe beleuchtet wird, *I* die Intensität des Fluoreszenzverhinderungs- bzw. Stimulationslichts am Maximum der Intensitätsverteilung und $I_S(\lambda)$ eine farbstoffspezifische Sättigungsintensität. Diese Sättigungsintensität ist üblicherweise dadurch definiert, dass das Beleuchten des Farbstoffs mit Fluoreszenzverhinderungslicht der Intensität $I = I_S$ die Photonenzahl oder Intensität des vom Farbstoff emittierten Fluoreszenzlichts um den Faktor 2 reduziert.

Das Ausbilden der Intensitätsverteilungen ist dabei nicht als abgeschlossener Schritt zu verstehen, nach dessen Beendigung die Intensitätsverteilungen wieder aufgehoben sind; vielmehr ist er so zu verstehen, dass die Intensitätsverteilungen auch während des nachfolgenden Abtastschritts ausgebildet bleiben.

5. Abtasten der Probe oder eines Ausschnitts der Probe mit der zuvor ausgebildeten, ein lokales Intensitätsminimum aufweisenden Intensitätsverteilung des Fluoreszenzverhinderungslichts an einer Abfolge von Abtastpositionen, wobei an jeder Abtastposition eine Intensität oder eine Photonenzahl von Fluoreszenzlicht detektiert und der Abtastposition zugeordnet wird. Dabei erfolgt das Abtasten bevorzugt entlang eines regelmäßigen, insbesondere eines kartesischen oder eines hexagonalen Gitters; dies ist allerdings nicht zwingend erforderlich. Insbesondere kann das Abtasten auch adaptiv erfolgen, beispielsweise, indem die Probe oder der Ausschnitt der Probe entlang eines Rasters mit einer relativ großen Schrittweite abgetastet wird und nur in denjenigen Bereichen, in denen eine Fluoreszenzemission

detektiert wird, das Abtasten mit einer kleineren, gegebenenfalls auf die Struktur angepassten Schrittweite erfolgt. Wenn die die Struktur aufweisenden Bereiche vorab identifiziert werden können (beispielsweise aus einem Epifluoreszenzbild der Probe oder des Ausschnitts der Probe), so kann auf das Abtasten außerhalb der dieser Bereiche auch vollständig verzichtet werden.

6. Erzeugen eines hochaufgelösten Rasterbildes der Struktur aus den einander zugeordneten Photonenzahlen oder Intensitäten des Fluoreszenzlichts und den Abtastpositionen, wobei jedem Bildpixel des Rasterbildes ein Helligkeitswert zugeordnet wird. Im einfachsten Fall besteht zwischen Abtastpositionen einerseits und Bildpixeln des Rasterbildes andererseits eine direkte eins-zu-eins-Beziehung, d. h. jedem Bildpixel wird die an der korrespondierenden Abtastposition detektierte Photonenzahl oder der (digitalisierte) Intensitätswert als Helligkeitswert direkt zugeordnet. Die Anzahlen der Abtastpositionen und der Bildpixel müssen allerdings nicht zwingend übereinstimmen, eine Zusammenfassung der detektierten Photonenzahlen oder Intensitäten von mehreren Abtastpositionen zu einem Bildpunkt ist ebenso möglich wie eine Interpolation von Bildpixeln aus den Photonenzahlen oder Intensitäten benachbarter Abtastpositionen.

Zur Steigerung des Dynamikumfangs des Rasterbildes, zur Hervorhebung schwach fluoreszierender Strukturen oder zur Anpassung des Helligkeitswerts an die Darstellungscharakteristik eines Anzeigegeräts oder die Wahrnehmungscharakteristik des menschlichen Auges kann der Helligkeitswert optional eine (monotone) Funktion der Photonenzahl oder des Intensitätswerts sein, beispielsweise in Form einer Logarithmus- oder Gammafunktion.

In einer bevorzugten Ausführungsform des Verfahrens weist das Fluoreszenzverhinderungslicht konkret eine toroidale Intensitätsverteilung mit einem lokalen Intensitätsminimum in Form einer zentralen Nullstelle auf, während mit dem Anregungslicht ein typischerweise beugungsbegrenzter, gaußförmiger Fokus ausgebildet wird dergestalt, dass das Minimum des Fluoreszenzverhinderungslichts und das Maximum des Anregungslichts räumlich zusammenfallen. Verfahren zur Ausbildung einer solchen toroidalen Intensitätsverteilung sind dem Fachmann aus dem Stand der Technik bekannt, beispielhaft sei an dieser Stelle nur die Anordnung einer helikalen Phasenplatte (Vortex-Phasenplatte) im Lichtstrahl des Fluoreszenzverhinderungslichts genannt. Die Intensitätsverteilungen des Anregungs- und Fluoreszenzverhinderungslichts sind in dieser Ausführungsform im Wesentlichen komplementär zueinander, d.h. an Stellen hoher Intensität des Anregungslichts ist die Intensität des Fluoreszenzverhinderungslichts gering und umgekehrt.

In einer parallelisierten Variante dieser Ausführungsform können alternativ mehrere, gegebenenfalls sogar sehr viele entsprechende Intensitätsverteilungen von Anregungs- und Fluoreszenzverhinderungslicht durch paarweise Interferenz von vier Anregungs- und Fluoreszenzverhinderungslichtstrahlen unter Ausbildung je zweier, zueinander orthogonaler stehender Wellen erzeugt werden [siehe "Nanoscopy with more than 100,000 'doughnuts'", A. Chmyrov et al. in Nature Meth. 10, 737 (2013)]. Dabei sind die stehenden Wellen des Anregungs-und des Fluoreszenzverhinderungslichts gegeneinander (phasen-)verschoben, so dass auch hier im Wesentlichen komplementäre Intensitätsverteilungen resultieren.

Zur Positionierung der Intensitätsverteilungen von Anregungs- und Fluoreszenzverhinderungslicht an den Abtastpositionen kann eine im Strahlengang angeordnete Strahlablenkvorrichtung genutzt werden, die beispielsweise mit Galvospiegeln realisiert werden kann. Für höhere Abtastgeschwindigkeiten und insbesondere unregelmäßig angeordnete Abtastpositionen eignen sich alternativ elektrooptische oder akustooptische Deflektoren, die ohne bewegliche Teile auskommen und eine besonders schnelle Ablenkung der Lichtstrahlen erlauben.

Die Detektion des Fluoreszenzlichts kann dabei abhängig von der Art der Intensitätsverteilungen von Anregungs- und Fluoreszenzverhinderungslicht mit einem Punktdetektor, einem Detektorarray bzw. einer Kamera erfolgen. Aufgrund ihrer Sensitivität besonders geeignet sind im Photonenzählmodus betriebene Avalanche-Fotodioden oder Avalanche-Fotodiodenarrays, aber auch (Hybrid)-Fotomultiplier oder integrierende Detektoren wie CCD- und sCMOS-Kameras. Im Falle einer punktförmigen Beleuchtung mit Anregungslicht und der Detektion mittels eines Punkt-Detektors, wird dieser zur Unterdrückung von Streu- und Hintergrundlicht bevorzugt hinter einer konfokalen Lochblende angeordnet. Aber auch im Falle einer punktförmigen Beleuchtung kann das Fluoreszenzlicht vorteilhaft mit einem Detektorarray registriert werden.

Das Beleuchten der Probe mit Anregungs- und Fluoreszenzverhinderungslicht und das Detektieren der Fluoreszenz an den Abtastpositionen kann bei Bedarf wiederholt werden, beispielsweise um das Signal-/Rauschverhältnis zu verbessern oder um Zeitreihen aufzunehmen. Gegebenenfalls können vor dem erneuten Abtasten weitere Farbstoffmoleküle fotoaktiviert werden, beispielsweise, um während des Abtastens ausgeblichenen Farbstoff zu ersetzen. Alternativ kann es gewünscht sein, eine ganz andere Teilmenge des Fluoreszenzfarbstoffs zu aktivieren. In diesem Fall müssen die (restlichen) fluoreszenten Farbstoffmoleküle zunächst wieder deaktiviert werden, was im einfachsten Fall durch Ausbleichen mit intensivem Anregungslicht bewirkt werden kann.

Bei der Erfindung handelt es sich weiter um ein Verfahren zur Lokalisation einzelner Moleküle eines Fluoreszenzfarbstoffs in einer Probe. Das Verfahren umfasst eine Fotoaktivierung einzelner Moleküle eines Fluoreszenzfarbstoffs in einer zwei Reaktionsschritte umfassenden Aktivierungsreaktion, eine Abtastung dieser Moleküle mit einer Intensitätsverteilung eines Anregungslichts und einer Intensitätsverteilung des Fluoreszenzverhinderungslichts sowie einer Fluoreszenzdetektion an jeder Abtastposition. Bei dieser Abtastung kann eine der beiden Intensitätsverteilungen ortsfest bleiben, mindestens eine der beiden Intensitätsverteilungen wird sequenziell an einer Mehrzahl von Abtastpositionen positioniert. Aus den Fluoreszenzintensitäten und den Abtastpositionen werden gegenüber vorhergehenden Positionsschätzwerten neue Positionsschätzwerte der Fluoreszenzmoleküle berechnet. Bei den neuen Positionsschätzwerten handelt es sich, wenn ruhende Fluoreszenzfarbstoffmoleküle lokalisiert werden, um verbesserte Positionsschätzwerte, d. h. solche, die die tatsächliche Position des betreffenden Fluoreszenzfarbstoffmoleküls mit geringerer Unsicherheit abschätzen. Werden sich bewegende Fluoreszenzfarbstoffmoleküle beobachtet, kann es sich um Werte handeln, die jeweils eine neue, geänderte Position des betreffenden Fluoreszenzfarbstoffmoleküls abschätzen. Die Schritte des Verfahrens sind im Einzelnen wie folgt:

1. Auswahl eines Fluoreszenzfarbstoffs, der so beschaffen ist, dass er initial in einer geschützten, nicht-fluoreszenten Form vorliegt und der durch Beleuchten mit Aktivierungslicht aus der geschützten in eine aktivierte, fluoreszente Form überführt, d. h. fotoaktiviert werden kann. Erfindungsgemäß wird der Fluoreszenzfarbstoff so ausgewählt, dass das Fotoaktivieren mindestens zwei jeweils lichtinduzierte Reaktionsschritte umfasst.

Das Verfahren umfasst weiter eine erste Gruppe von Verfahrensschritten:
2. Fotoaktivieren eines oder mehrerer, räumlich einen Mindestabstand *d* voneinander entfernter Moleküle des Fluoreszenzfarbstoffs aus der geschützten, nicht-fluoreszenten Form in die aktivierte, fluoreszente Form durch Beleuchten der Probe mit Aktivierungslicht. Erfindungsgemäß erfolgt das Fotoaktivieren, wie auch beim erstgenannten erfindungsgemäßen Verfahren, durch eine mindestens zwei lichtinduzierte Reaktionsschritte umfassende Aktivierungsreaktion, wobei der Fluorophor erst nach dem letzten der Reaktionsschritte ausgebildet wird und der Farbstoff seine fluoreszenten Eigenschaften erhält. Zu den Anforderungen an und die Ausgestaltung des Fotoaktivierungsprozesses gilt das in der Beschreibung des ersten erfindungsgemäßen Verfahrens Gesagte.

Um den Mindestabstand *d* zwischen mehreren aktivierten Farbstoffmolekülen zu gewährleisten, muss die räumliche Dichte von aktivierten Farbstoffmolekülen beim Beleuchten mit Aktivierungslicht genau kontrolliert werden. Hierzu können die Beleuchtungsparameter des Aktivierungslichts, also insbesondere die Beleuchtungsdauer und -intensität, einmalig durch Versuch und Irrtum so bestimmt werden, dass die Anforderung des Vorliegens räumlich isolierter Farbstoffmoleküle für eine gegebene Probe und unter gegebenen Aufnahmebedingungen sichergestellt ist. Wenn die Probe während der Fotoaktivierung kontinuierlich beobachtet werden kann, z. B. indem sie mit Anregungslicht beaufschlagt wird, wobei Fluoreszenzlicht detektiert wird, so kann die Fotoaktivierung aber auch unter direkter Kontrolle durchgeführt und die Beleuchtung mit Aktivierungslicht bei Erreichen eines Grenzwerts der Moleküldichte gestoppt werden. Erfolgt die Aktivierung in einem lokal eng begrenzten Bereich, kann die Aktivierung dann gestoppt werden, wenn bei der Beobachtung ein Fluoreszenzsignal einen Schwellwert, der auch 0 (null) sein kann, überschritten wird. Die kontrollierte Aktivierung ist besonders dann vorteilhaft, wenn im Laufe der Datenaufnahme die Gesamtmenge an Farbstoff infolge von Fotobleichen abnimmt und die Beleuchtungsdauer oder -intensität erhöht werden muss, um eine konstante Dichte von aktivierten Farbstoffmolekülen aufrecht zu erhalten oder um in einem lokal begrenzten Bereich ein Farbstoffmolekül zu aktivieren. Der Abstand zwischen aktivierten Farbstoffmolekülen kann alternativ oder zusätzlich dadurch gesteuert werden, dass die Probe nicht großflächig und homogen mit Aktivierungslicht beleuchtet wird, sondern mit einem Beleuchtungsmuster, beispielsweise in Form von Beleuchtungspunkten auf einem Gitter.

3. Bestimmen initialer Positionsschätzwerte eines oder mehrerer aktivierter Farbstoffmoleküle als Startwerte für den nachfolgenden Abtast- und Detektionsschritt des Verfahrens. Die initialen Positionsschätzwerte werden derart bestimmt, dass sie höchstens eine Unsicherheit von *d*/2 aufweisen, so dass sie einem der mindestens den Abstand *d* voneinander entfernten, aktivierten Farbstoffmoleküle eindeutig zugeordnet werden können.

Die initialen Positionsschätzwerte lassen sich grundsätzlich auf verschiedene Arten ermitteln. Insbesondere können die Positionsschätzwerte aus einem Fluoreszenzbild der Probe bzw. eines Ausschnitts der Probe durch Lokalisation der aktivierten Farbstoffmoleküle bestimmt werden. Hierzu eignet sich insbesondere ein mit einer Kamera oder mittels konfokalem Laserscanning aufgenommenes Fluoreszenzbild. In einer spezielleren Variante des Verfahrens können die initialen Positionsschätzwerte auch mittels STED-Mikroskopie bestimmt werden, was gegebenenfalls eine höhere räumliche Dichte aktivierter Farbstoffmoleküle ermöglicht.

Die Bestimmung der initialen Positionsschätzwerte mittels konfokaler oder STED-Mikroskopie kann auch dahingehend variiert werden, dass nicht ganze Bildfelder abgescannt werden, um (nachträglich) in diesen Bildern einzelne Farbstoffmoleküle zu identifizieren und zu lokalisieren, sondern dass die Probe abgetastet wird, bis ein einzelnes, aktiviertes Farbstoffmolekül detektiert wird und dessen Position näherungsweise aus der jeweiligen Scanposition bestimmt werden kann. Der nachfolgende Abtast- und Detektionsschritt kann sich dann jeweils unmittelbar an die Detektion eines Farbstoffmoleküls anschließen, so dass diese Verfahrensschritte nicht notwendigerweise streng voneinander getrennt durchgeführt werden müssen, sondern auch abwechselnd erfolgen können. Ebenso kann auch die Fotoaktivierung mit dem Abtasten gekoppelt werden. Hierzu kann die Probe Punkt für Punkt und Zeile für Zeile mit Aktivierungs- und Anregungslicht (gleichzeitig oder als schnelle Sequenz) abgetastet werden, bis ein einzelnes, aktiviertes Farbstoffmolekül detektiert wird. Der Abtast- und Detektionsschritt kann sich wieder unmittelbar an die Detektion eines Farbstoffmoleküls anschließen.

Insbesondere wenn die Aktivierung in einem lokal eng begrenzten Bereich mittels eines beugungsbegrenzt fokussierten Aktivierungslichtstrahls erfolgt, kann das Bestimmen des initialen Positionsschätzwerts auch unmittelbar aus der Position des bekannten Fokuspunkts des Aktivierungslichtstrahls erfolgen; diese Position wird dann als initialer Positionsschätzwert genutzt.

4. Ausbilden einer Intensitätsverteilung eines Anregungslichts und einer Intensitätsverteilung eines Fluoreszenzverhinderungslichts in der Probe. Das Anregungslicht ist seiner Wellenlänge nach geeignet, den aktivierten Fluoreszenzfarbstoff zur Fluoreszenzemission anzuregen, während unter dem Begriff des Fluoreszenzverhinderungslichts wiederum jede Art von Licht verstanden werden soll, die geeignet ist, die Fluoreszenz aktivierter, fluoreszenter Farbstoffmoleküle bei der Beleuchtung mit Anregungslicht zu verhindern, zu reduzieren oder gänzlich zu unterdrücken. Konkret kann das Fluoreszenzverhinderungslicht Stimulationslicht sein, das eine stimulierte Emission elektronisch angeregter Farbstoffmoleküle induziert, wodurch die Farbstoffmoleküle (zurück) in den elektronischen Grundzustand überführt und an einer spontanen Fluoreszenzemission gehindert werden. In der Regel bleiben beide Intensitätsverteilungen - zumindest aber die Intensitätsverteilung des Anregungslichts - auch während des nachfolgenden Abtastschritts ausgebildet.

Erfindungsgemäß weist zumindest das Fluoreszenzverhinderungslicht eine Intensitätsverteilung mit einem lokalen Intensitätsminimum auf und verhindert oder unterdrückt die Emission von Fluoreszenz in Bereichen außerhalb dieses lokalen Intensitätsminimums. Dabei steht nicht die Reduzierung des Volumens einer effektiven PSF im Sinne einer Auflösungserhöhung wie bei der STED-Mikroskopie im Vordergrund, vielmehr geht es vorrangig darum, unerwünschte Fluoreszenz von Farbstoffmolekülen aus fokalen Randbereichen oder anderen Ebenen der Probe zu unterdrücken. Dadurch kann die Detektion von Fluoreszenz auf ein in der Nähe des lokalen Intensitätsminimums befindliches, fluoreszentes Farbstoffmolekül beschränkt werden, und unerwünschte Beiträge von anderen fluoreszenten Farbstoffmolekülen, insbesondere aus fokalen Randbereichen oder anderen Ebenen der Probe, können unterdrückt werden. Anders als in der STED-Mikroskopie kann es daher mit Blick auf eine bestimmte Ausführungsform der nachfolgenden Abtast- und Lokalisationsschritte sogar ausdrücklich erwünscht sein, das Intensitätsminimum möglichst breit und den Intensitätsanstieg in der Nähe des Intensitätsminimums möglichst flach zu gestalten, um so die Fluoreszenzemission eines in der Nähe des Intensitätsminimums befindlichen fluoreszenten Farbstoffmoleküls bei einer Verlagerung des Intensitätsminimums nur wenig zu beeinflussen. Eine derartige Intensitätsverteilung mit breiter Nullstelle kann beispielsweise durch Phasenmodulation des Fluoreszenzverhinderungslichtstrahls mit einer Vortex-Phasenplatte höherer Ordnung erzeugt werden, deren Phasenverzögerung nicht von 0 bis $2\pi$ mit dem Umlaufwinkel variiert (wie in der STED-Mikroskopie üblich), sondern von 0 bis $4\pi$ oder einem höheren Vielfachen von $2\pi$. Ungeachtet des zuvor Gesagten kann eine Auflösungserhöhung durch das Fluoreszenzverhinderungslicht im Sinne der STED-Mikroskopie in einzelnen Ausführungsformen des Verfahrens aber auch vorteilhaft und erwünscht sein.

In Abhängigkeit von der jeweiligen Ausführungsform des Verfahrens kann die Intensitätsverteilung des Anregungslichts in unterschiedlicher Weise ausgebildet sein, insbesondere kann aber auch die Intensitätsverteilung des Anregungslichts ein lokales Intensitätsminimum aufweisen. Bevorzugte Ausführungsformen des Verfahrens umfassen die Kombination von punktförmig fokussiertem Anregungslicht mit einer toroidalen Intensitätsverteilung von Fluoreszenzverhinderungslicht und die Kombination zweier toroidaler Intensitätsverteilungen von Anregungs- und Fluoreszenzverhinderungslicht.

Das Verfahren umfasst schließlich eine zweite Gruppe von Verfahrensschritten:

5. Abtasten der Probe oder eines Ausschnitts der Probe mit einer der zuvor ausgebildeten, ein Intensitätsminimum aufweisenden Intensitätsverteilungen an einer Abfolge von Abtastpositionen, wobei an jeder Abtastposition eine Photonenzahl oder eine Intensität von Fluoreszenzlicht detektiert und der Abtastpositionen zugeordnet wird. Diese aus den Photonenzahlen oder Fluoreszenzintensitäten und den Abtastpositionen gebildeten Wertepaare bilden die Datenbasis für den nachfolgenden Lokalisationsschritt. Die Abtastpositionen sind so gewählt, dass sich im Abstand von weniger als *d*/2 um den Positionsschätzwert jedes zu lokalisierenden Farbstoffmoleküls mindestens zwei Abtastpositionen befinden. Das Abtasten erfolgt dabei mit einer Intensitätsverteilung, die ein lokales Intensitätsminimum - idealerweise eine Intensitätsnullstelle - aufweist. Diese kann eine Intensitätsverteilung von Anregungslicht oder von Fluoreszenzverhinderungslicht sein. In jedem Falle wird die Probe oder der Ausschnitt der Probe bei jedem Abtastschritt mit Anregungslicht beaufschlagt. Erfolgt die Abtastung mit Fluoreszenzverhinderungslicht, so kann die Intensitätsverteilung des Anregungslichts ortsfest sein. Handelt es sich bei dem Fluoreszenzverhinderungslicht um Stimulationslicht, so wird die Probe oder der Ausschnitt der Probe in jedem Abtastschritt mit Fluoreszenzverhinderungslicht, also dem Stimulationslicht beaufschlagt. Handelt es sich bei dem Fluoreszenzverhinderungslicht um ein Schaltlicht, das Fluorophore in einen (meta-)stabilen, nicht-fluoreszenten Zustand schaltet, kann es ausreichen, die Probe zu Beginn des Abtastens einmalig mit Fluoreszenzverhinderungslicht zu beaufschlagen. Insofern ist der erste Schritt der zweiten Gruppe von Verfahrensschritten nicht streng getrennt vom letzten Schritt der ersten Gruppe. Insbesondere bei der Anwendung hoher Intensitäten des das Intensitätsminimum ausbildenden Lichts entsteht ausgehend von dem Intensitätsminimum einer steiler Intensitätsgradient, so dass sich die Fluoreszenzemission eines nahe des Intensitätsminimums befindlichen Farbstoffmoleküls schon bei geringer Verlagerung der Abtastposition stark verändert. Dabei kann die Fluoreszenzemission mit zunehmendem Abstand des Farbstoffmoleküls vom Intensitätsminimum zunehmen (wenn die Abtastung mit Anregungslicht erfolgt) oder abnehmen (wenn die Abtastung mit Fluoreszenzverhinderungslicht erfolgt). Die starke Abhängigkeit der Fluoreszenzemission von der Abtastposition bildet unabhängig von ihrer Richtung die Grundlage für die Verbesserung der Positionsschätzung im Lokalisationsschritt.

In einer bevorzugten Ausführungsform weisen sowohl das Anregungs- als auch das Fluoreszenzverhinderungslicht Intensitätsverteilungen mit räumlich überlagerten Intensitätsminima auf, beispielsweise in Form toroidaler (donutförmiger) Intensitätsverteilungen. Die Abtastung erfolgt in dieser Ausführungsform weiter bevorzugt mit der Intensitätsverteilung des Anregungslichts, während die Position der Intensitätsverteilung des Fluoreszenzverhinderungslichts während der Abtastung an den Abtastpositionen, die je einem aktivierten fluoreszentem Farbstoffmolekül zugeordnet sind, ortsfest ist und somit auf die Änderung des Fluoreszenzsignals an den verschiedenen Abtastpositionen keinen Einfluss hat. Gleichwohl kann auch die Intensitätsverteilung des Fluoreszenzverhinderungslichts beim Abtasten beim Übergang von einem Farbstoffmolekül zu einem anderen mit verlagert werden. Für das Fluoreszenzverhinderungslicht eignen sich insbesondere Intensitätsverteilungen, die innerhalb des Abtastbereichs eines Farbstoffmoleküls eine nur geringe Intensitätsvariation aufweisen und die beispielsweise mit Vortex-Phasenplatten höherer Ordnung erzeugt werden können (siehe oben). Der Zweck des Fluoreszenzverhinderungslichts besteht in dieser Ausführungsform ausschließlich darin, die Beiträge zur detektierten Fluoreszenz von Farbstoffmolekülen außerhalb der Intensitätsminima zu unterdrücken und so die Lokalisation der abgetasteten Moleküle zu ermöglichen bzw. durch Eliminierung von Störbeiträgen präziser zu machen. Prinzipiell ist auch ein Abtasten mit beiden Intensitätsverteilungen möglich, dann ist allerdings zu berücksichtigen, dass die Fluoreszenz der Farbstoffmoleküle beim Abtasten durch beide Intensitätsverteilungen beeinflusst wird. Vorteile können sich insbesondere ergeben, wenn Fluoreszenzanregung, -unterdrückung mit Stimulationslicht und -detektion unter Nutzung zeitlicher Gates erfolgt, insbesondere wenn die Fluoreszenzdetektion auf ein sehr kurzes Zeitfenster, beispielsweise auf weniger als die Hälfte, bevorzugt weniger als ein Viertel, weiter bevorzugt auf weniger als ein Zehntel der Fluoreszenzlebensdauer nach dem Zeitpunkt, zu dem zum zeitlichen Maximum des Anregungspulses aus dem Beobachtungsvolumen emittiertes Fluoreszenzlicht auf dem Detektor ein Signal erzeugen würde, beschränkt wird.

In einer weiteren bevorzugten Ausführungsform wird die aus der STED-Mikroskopie bekannte Kombination aus einem gaußförmig fokussierten Lichtfleck von Anregungslicht mit einer toroidalen (donutförmigen) Intensitätsverteilung von Fluoreszenzverhinderungslicht verwendet. Hier weist nun nur das Fluoreszenzverhinderungslicht eine Intensitätsverteilung mit einem Intensitätsminimum auf, und die Abtastung erfolgt entsprechend mit dem Fluoreszenzverhinderungslicht, während die Position der Intensitätsverteilung des Anregungslichts während der Abtastung an den Abtastpositionen, die je einem aktivierten fluoreszentem Farbstoffmolekül zugeordnet sind, bevorzugt ortsfest ist und somit auf die Änderung des Fluoreszenzsignals an den verschiedenen Abtastpositionen keinen Einfluss hat. Die Intensitätsverteilung des Anregungslichts wird während des Abtastens nur beim Übergang von einem Farbstoffmolekül zu einem anderen oder beim Verfolgen der Bewegung eines Farbstoffmoleküls in der Probe mitgeführt.

Die Mindestanzahl von zwei Abtastpositionen pro zu lokalisierendem Farbstoffmolekül erlaubt die hochgenaue Lokalisation des Farbstoffmoleküls in einer Dimension. Ist dagegen eine Lokalisation in zwei Dimensionen gewünscht, so erhöht sich die Mindestzahl von Abtastpositionen auf drei pro Farbstoffmolekül. Dabei ist eine Anordnung der Abtastpositionen vorteilhaft, bei der sich das Farbstoffmolekül innerhalb des aus den drei Abtastpositionen aufgespannten Dreiecks befindet; dies ist allerdings nicht strikt erforderlich. Für eine Lokalisation in drei Dimensionen erhöht sich die minimale Anzahl auf vier Abtastpositionen pro Farbstoffmolekül. Auch hier ist eine Anordnung der Abtastpositionen vorteilhaft (aber nicht zwingend), bei der sich das Farbstoffmolekül innerhalb eines durch vier Abtastpositionen aufgespannten Tetraeders befindet. In der Praxis kann eine Erhöhung der Anzahl von Abtastpositionen über diese Mindestanzahlen hinaus sinnvoll sein, um die Lokalisationsgenauigkeit weiter zu verbessern. Insbesondere kann es vorteilhaft sind, als eine Abtastposition den vor der jeweiligen Durchführung der zweiten Gruppe bestimmten Positionsschätzwert zu verwenden. Hierdurch wird erreicht, dass aus den registrierten Fluoreszenzsignalen eindeutig ein Positionsschätzwert erhalten wird, d. h. dass die gemessenen Fluoreszenzsignale nicht doppeldeutig sein können.

6. Im abschließenden Lokalisationsschritt werden aus den einander zugeordneten Photonenzahlen oder Intensitäten und Abtastpositionen verbesserte Positionsschätzwerte der aktivierten Farbstoffmoleküle ermittelt. Im einfachsten Fall erfolgt dies durch Bilden der mit den Photonenzahlen bzw. Fluoreszenzintensitäten gewichteten Summe der Ortsvektoren der Abtastpositionen. Weitere gängige, weitaus genauere Verfahren bedienen sich beispielsweise eines *Maximum Likelihood Estimators* (MLE) und sind aus dem Stand der Technik zur MINFLUX-Nanoskopie bekannt.

Erfindungsgemäß ist der Wert von d so zu wählen, dass den aktivierten fluoreszenten Farbstoffmolekülen initiale Positionsschätzwerte eindeutig zugeordnet werden können und dass das detektierte Fluoreszenzlicht an jeder Abtastposition nur von jeweils einem einzigen aktivierten Molekül des Fluoreszenzfarbstoffs stammt. Dies bedeutet einerseits, dass der Mindestabstand zwischen den aktivierten Farbstoffmolekülen das optische Auflösungsvermögen des Verfahrens, mit dem die initialen Positionsschätzwerte ermittelt werden, nicht unterschreiten darf somit aufgrund der optischen Beugungsgrenze in der Regel $d \geq 250$ nm vorgegeben ist. Nur in dem Fall, dass die initialen Positionsschätzwerte mit einem bereits höher auflösenden Verfahren (beispielsweise durch STED-Mikroskopie) bestimmt werden,

kann der Wert von Wert von d gegebenenfalls auch kleiner gewählt werden. Dabei ist aber auch zu berücksichtigen, dass der Mindestabstand zwischen den aktivierten Farbstoffmolekülen auch dadurch zu kleinen Werten hin beschränkt ist, dass beim Abtasten der Probe oder des Ausschnitts der Probe mit der ein Intensitätsminimum aufweisen Lichtverteilung jeweils nur ein einziges aktiviertes Molekül des Fluoreszenzfarbstoffs zum detektierten Fluoreszenzsignal beitragen darf. Es ist daher nur in speziellen Varianten des Verfahrens - beispielsweise beim Abtasten mit einer Kombination aus einem gaußförmig fokussierten Lichtfleck von Anregungslicht und einer toroidalen (donutförmigen) Intensitätsverteilung von Fluoreszenzverhinderungslicht-möglich, den Wert von d auf unter 250 nm zu reduzieren.

Bei der Implementierung des erfindungsgemäßen Verfahrens muss die Abfolge von Abtastpositionen nicht notwendigerweise vollständig vor Beginn des Abtastens festgelegt werden, sondern kann auch sukzessive ergänzt werden. In besonders vorteilhafter Weise kann die Abfolge von einem Farbstoffmolekül zugeordneten Abtastpositionen unter Berücksichtigung des oder der vorangegangenen Bestimmungsschritts oder Bestimmungsschritte fortgesetzt werden. Da die Unsicherheit in der Positionsschätzung durch einen Lokalisationsschritt erheblich verkleinert wird, können Abtastpositionen nachfolgend sehr viel dichter um das zu lokalisierende Farbstoffmolekül angeordnet werden. Die Abtastung an diesen neu festgelegten Abtastpunkten führt zusammen mit einer (weiteren) Erhöhung der Intensität des Lichts, mit dem die Abtastung erfolgt, zur Verbesserung der Positionsschätzungen im nächsten Bestimmungsschritt. Eine wiederholte Anwendung dieser Schritte erlaubt eine Lokalisation des Farbstoffmoleküls bis in den Bereich weniger Nanometer.

In einer Weiterbildung des erfindungsgemäßen Verfahrens erfolgt die Bestimmung von Positionsschätzwerten eines einzelnen aktivierten Farbstoffmoleküls mehrfach, beispielsweise in festen Intervallen. Wird das Verfahren durchgeführt, um die Bewegung eines Farbstoffmoleküls in einer Probe zu verfolgen, so werden die Abtastpositionen für einen Bestimmungsschritt, wie dies auch im Falle der Lokalisation nicht bewegter Farbstoffmoleküle der Fall sein kann, anhand des Positionsschätzwerts des vorangehenden Bestimmungsschritts bestimmt. Bei der Verfolgung von Molekülen werden vorteilhaft die Abstände der Abtastpositionen angepasst an die Geschwindigkeit und die Art der Bewegung der Farbstoffmoleküle, insbesondere soweit diese aus den vorangehenden Bestimmungsschritten bekannt sind. Ist die Bewegung schnell und zufällig, so werden große Abstände der Abtastpositionen gewählt, damit das Molekül jeweils zuverlässig innerhalb des durch die Abtastpositionen festgelegten Bereichs, in dem die Position eines Moleküls geschätzt werden kann, liegt. Ist die Bewegung dagegen beispielsweise langsam und gerichtet, so werden die Abstände der Abtastpunkte kleiner, aber ebenfalls so gewählt, dass Molekül jeweils zuverlässig innerhalb des durch die Abtastpositionen festgelegten Bereichs, in dem die Position eines Moleküls geschätzt werden kann, liegt. In beiden Fällen wird das Zentrum der Menge der Abtastpositionen an den Ort verschoben, an dem das Molekül während der nächsten Abfolge von Abtastschritten erwartet wird. Bei einer zufälligen Bewegung ist dies der Ort, der dem zuletzt bestimmten Positionsschätzwert entspricht. Aus den aufeinanderfolgend bestimmten Positionsschätzwerten kann eine Trajektorie des Farbstoffmoleküls rekonstruiert, visualisiert und ggf. weiter analysiert werden. Bei Verwendung des Farbstoffs als Marker für ein Biomolekül, beispielsweise ein Protein oder ein Lipid, eignen sich derartige Trajektorien zur Untersuchung von dynamischen zellulären Prozessen, an denen das markierte Biomolekül beteiligt ist. Neben der hohen räumlichen Auflösung erlaubt das erfindungsgemäße Verfahren auch eine erheblich schnellere Bestimmung der Positionen einzelner Moleküle als es mit den aus dem Stand der Technik bekannten Methoden möglich ist und erweitert so die Anwendbarkeit der Einzelmolekül-Verfolgung auf schnelle dynamische Prozesse.

Der gesamte Prozess der Verfolgung einzelner Farbstoffmoleküle kann für weitere Farbstoffmoleküle wiederholt werden. Eine solche Wiederholung kann mit dem Aktivieren beginnen. Befinden sich nach der Verfolgung eines Moleküls andere in einem aktivierten Zustand, kann es ausreichen, nur die zweite Gruppe von Verfahrensschritten, jetzt an einem weiteren Farbstoffmolekül, zu wiederholen. Auch aus der Menge von Trajektorien kann schließlich ein hochaufgelöstes Abbild einer Struktur in der Probe rekonstruiert werden. Beispielweise können Werte von Diffusionskonstanten lokal aufgelöst bestimmt werden. Die räumliche Verteilung dieser Werte kann dann eine Struktur abbilden.

In einer anderen Weiterbildung des erfindungsgemäßen Verfahrens werden die Lokalisationen genutzt, um ein hochaufgelöstes Abbild einer mit dem Fluoreszenzfarbstoff markierten Struktur in der Probe zu rekonstruieren, beispielsweise in Form eines zweidimensionalen Histogramms. Diese Art der Bildrekonstruktion ist aus der STORM- und PALM-Mikroskopie bekannt. Um eine hochaufgelöste, räumlich möglichst kontinuierliche Abbildung der Struktur generieren zu können ist es allerdings erforderlich, dass die Positionen einer hinreichend großen Zahl von Fluoreszenzfarbstoffmolekülen - typischerweise mehrerer Tausend - bestimmt wird. Hierzu werden die Verfahrensschritte vom Fotoaktivieren bis zur Lokalisierung der aktivierten Farbstoffmoleküle mehrfach angewendet, um die gewünschte hohe Zahl von Molekülen des Fluoreszenzfarbstoffs zu lokalisieren. Dabei müssen die jeweils aktiven Farbstoffmoleküle zwischen den Wiederholungen in einen nicht fluoreszenten Zustand überführt werden. Dies kann im einfachsten Fall dadurch erreicht werden, dass die aktiven Moleküle mit intensivem Anregungslicht irreversibel ausgebleicht werden. Sofern die Fotoaktivierung reversibel ist, können die aktivierten Moleküle auch mit Licht geeigneter Wellen in den nichtfluoreszenten Zustand zurückversetzt werden.

Bei der Erfindung handelt es sich schließlich um ein weiteres Verfahren zur Lokalisation einzelner Moleküle eines Fluoreszenzfarbstoffs in einer Probe, wobei das Verfahren Merkmale der ersten beiden erfindungsgemäßen Verfahren vereint. Die Schritte des Verfahrens sind im Einzelnen wie folgt:

1. Auswahl eines Fluoreszenzfarbstoffs, der so beschaffen ist, dass er initial in einer geschützten, nicht-fluoreszenten Form vorliegt und der durch Beleuchten mit Aktivierungslicht aus der geschützten in eine aktivierte, fluoreszente Form überführt, d. h. fotoaktiviert werden kann. Erfindungsgemäß wird der Fluoreszenzfarbstoff so ausgewählt, dass das Fotoaktivieren mindestens zwei jeweils lichtinduzierte Reaktionsschritte umfasst.

Das Verfahren umfasst weiter die Gruppe von Verfahrensschritten:

2. Fotoaktivieren eines oder mehrerer, räumlich einen Mindestabstand d voneinander entfernter Moleküle des Fluoreszenzfarbstoffs aus der geschützten, nicht-fluoreszenten Form in die aktivierte, fluoreszente Form durch Beleuchten der Probe mit Aktivierungslicht, wobei das Fotoaktivieren durch eine mindestens zwei lichtinduzierte Reaktionsschritte umfassende Aktivierungsreaktion erfolgt.

3. Ausbilden einer Intensitätsverteilung eines Anregungslichts und einer Intensitätsverteilung eines Fluoreszenzverhinderungslichts in der Probe, wobei die Intensitätsverteilung des Fluoreszenzverhinderungslichts ein lokales Intensitätsminimum aufweist. In der Regel bleiben beide Intensitätsverteilungen - zumindest aber die Intensitätsverteilung des Anregungslichts - auch während des nachfolgenden Abtastschritts ausgebildet.

4. Abtasten der Probe oder eines Ausschnitts der Probe mit der ein lokales Intensitätsminimum aufweisenden Intensitätsverteilung des Fluoreszenzverhinderungslichts an einer Abfolge von Abtastpositionen, die untereinander einen Abstand von nicht mehr als *d*/2 aufweisen, wobei an jeder Abtastposition eine Intensität oder eine Photonenzahl von Fluoreszenzlicht detektiert und der Abtastposition zugeordnet wird. Dabei erfolgt das Abtasten bevorzugt entlang eines regelmäßigen, insbesondere eines kartesischen oder eines hexagonalen Gitters; dies ist allerdings nicht zwingend. Insbesondere kann das Abtasten auch adaptiv erfolgen, beispielsweise, indem die Probe oder der Ausschnitt der Probe entlang eines Rasters mit einer relativ großen Schrittweite abgetastet wird und nur in denjenigen Bereichen, in denen eine Fluoreszenzemission detektiert wird, das Abtasten mit einer kleineren, gegebenenfalls auf die Struktur angepassten Schrittweite erfolgt. Wenn die die Struktur aufweisenden Bereiche vorab identifiziert werden können (beispielsweise aus einem Epifluoreszenzbild der Probe oder des Ausschnitts der Probe), so kann auf das Abtasten außerhalb der dieser Bereiche auch vollständig verzichtet werden.

5. Lokalisieren von aktivierten Farbstoffmolekülen aus den einander zugeordneten Photonenzahlen oder Intensitäten des Fluoreszenzlichts und den Abtastpositionen mit einer Unsicherheit von höchstens *d*/10 in mindestens einer Raumrichtung,

Erfindungsgemäß ist der Wert von d so zu wählen, dass den aktivierten fluoreszenten Farbstoffmolekülen initiale Positionsschätzwerte eindeutig zugeordnet werden können und dass das detektierte Fluoreszenzlicht an jeder Abtastposition nur von jeweils einem einzigen aktivierten Molekül des Fluoreszenzfarbstoffs stammt.

Weitere vorteilhafte Weiterbildungen der Erfindung ergeben sich aus den Patentansprüchen, der Beschreibung und den Zeichnungen. Die in der Beschreibung beschriebenen Vorteile von Merkmalen und von Kombinationen mehrerer Merkmale der Erfindung sind lediglich beispielhaft und können alternativ oder kumulativ zur Wirkung kommen, ohne dass die Vorteile zwingend von erfindungsgemäßen Ausführungsformen erzielt werden müssen. Ohne dass hierdurch der Gegenstand der beigefügten Patentansprüche verändert wird, gilt hinsichtlich des Offenbarungsgehalts der ursprünglichen Anmeldungsunterlagen und des Patents das Folgende: weitere Merkmale sind den Zeichnungen zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Figuren dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

Der in den Patentansprüchen und der Beschreibung genutzte unbestimmte Artikel "ein" für ein Merkmal ist so zu verstehen, dass es sich hinsichtlich der Anzahl um genau eine oder auch um mehrere Ausführungen dieses Merkmals handeln kann, ohne dass es einer expliziten Verwendung des Adverbs "mindestens" bedarf. Die in den Patentansprüchen angeführten Merkmale können gegebenenfalls durch weitere Merkmale ergänzt werden oder auch die einzigen Merkmale sein, die das jeweilige Verfahren aufweist.

## Kurzbeschreibung der Figuren

| | |
|---|---|
| Fig. 1 | zeigt einen Abschnitt eines erfindungsgemäßen Verfahrens in Form eines Ablaufdiagramms. |
| Fig. 2 | zeigt Fotoaktivierungs- und Abtastschritte in einem erfindungsgemäßen Verfahren. |
| Fig. 3 | zeigt einen Fluoreszenzfarbstoff zur Anwendung in den erfindungsgemäßen Verfahren. |
| Fig. 4 | illustriert eine Ausführungsform des Verfahrens mit Abtastpositionen entlang eines Gitters und einer direkten Bilderzeugung. |
| Fig. 5 | illustriert eine Ausführungsform des Verfahrens mit der Lokalisation einzelner Fluoreszenzfarbstoffmoleküle zur Erzeugung eines Bildes. |
| Fig. 6 | zeigt eine tabellarische Übersicht verschiedener Ausführungsformen der erfindungsgemäßen Verfahren. |

## Beschreibung der Figuren

In **Fig. 1** ist ein Abschnitt eines erfindungsgemäßes Verfahren in Form eines Ablaufdiagrammes dargestellt. Zunächst wird in einem Fotoaktivierungsschritt S1 ein Teil eines Fluoreszenzfarbstoffs, mit dem eine Struktur in einer Probe angefärbt ist, durch Beleuchten mit Aktivierungslicht aus einer geschützten, initial nicht fluoreszenten Form in eine aktivierte, fluoreszente Form überführt. Erfindungsgemäß erfolgt diese Fotoaktivierung durch mindestens zwei lichtinduzierte Reaktionsschritte. Im nachfolgenden Abtast- und Detektionsschritt S2 wird Anregungslicht, das den Fluoreszenzfarbstoff zur Abgabe von Fluoreszenzlicht anregt, und Fluoreszenzverhinderungslicht, das die Fluoreszenzabgabe durch den Fluoreszenzfarbstoff verhindert, reduziert oder gänzlich unterdrückt, an einer Abfolge von Abtastpositionen in der Probe positioniert (Positionierteilschritt S2.1). An jeder der Abtastpositionen, an denen die Probe beleuchtet wird, wird eine Photonenzahl oder eine Intensität von Fluoreszenzlicht detektiert (Abtast- und Detektionsteilschritt S2.2). Die detektierten Photonenzahlen oder Intensitäten des Fluoreszenzlichts werden zusammen mit der jeweiligen Abtastposition für die spätere Verarbeitung in einem Datenspeicher 1 abgelegt. Optional kann die Abtastung an allen oder an ausgewählten Abtastpositionen wiederholt werden. Ebenfalls optional können alle Verfahrensschritte, d.h. der Fotoaktivierungsschritt S1 und der Abtast- und Detektionsschritt S2 wiederholt werden, um einen weiteren oder einen anderen Teil des Fluoreszenzfarbstoffs, mit dem die Probe markiert ist, zu aktivieren und abzutasten.

Bei der in **Fig. 1** dargestellten Ausführungsform wird schließlich aus den an den Abtastpositionen detektierten Photonenzahlen oder Intensitäten von Fluoreszenzlicht ein hochaufgelöstes Bild 2 erzeugt. Das Bild 2 ist beispielweise ein Rasterbild, dessen Bildpixel die an jeder Abtastposition detektierten Fluoreszenzsignale wiedergeben und damit eine räumliche Repräsentation der mit dem Farbstoff markierten Struktur darstellen. Sofern die Fotoaktivierung so gesteuert wird, dass einzelne, räumlich voneinander getrennte Moleküle des Fluoreszenzfarbstoffs aktiviert werden, können die nun einzelnen Molekülen zuzuordnenden Photonenzahlen oder Intensitäten des Fluoreszenzlichts alternativ in einem (nicht gezeigten) Zwischenschritt zur Lokalisation dieser Moleküle des Fluoreszenzfarbstoffs genutzt und das Bild 2 aus den Positionen der lokalisierten Farbstoffmoleküle rekonstruiert werden.

**Fig. 2** zeigt einen Ausschnitt 3 einer Probe, die eine Struktur 4 enthält. Die Struktur 4 ist mit einem Fluoreszenzfarbstoff 5 markiert, der zunächst in einer geschützten, nicht fluoreszenten Form 6 vorliegt. Im einleitenden Fotoaktivierungsschritt S1 wird der Ausschnitt 3 der Probe mit Aktivierungslicht 7 beleuchtet, das einen kleinen Teil des Fluoreszenzfarbstoffs 5 in eine aktivierte, fluoreszente Form 8 überführt. Die Fotoaktivierung 9 erfolgt in einer zweistufigen Reaktion 10, hier durch die Reaktionsschritte 11 in Form der Abspaltung 12 zweier fotolabiler Schutzgruppen 13 und 14, wodurch der aktivierte, fluoreszente Farbstoff 5, 8 gebildet wird. Nach der Fotoaktivierung 9 wird der Ausschnitt 3 der Probe mit Anregungslicht 16, hier in Form einer ein lokales Maximum aufweisenden Intensitätsverteilung 15, und mit einer ein lokales Minimum aufweisenden Intensitätsverteilung 17 von Fluoreszenzverhinderungslicht 18 an einer Abfolge von Abtastpositionen 19 abgetastet, von denen hier beispielhaft nur die ersten zwei Abtastpositionen gezeigt sind. Durch die erfindungsgemäße Ausgestaltung der Fotoaktivierung 9 in Form einer zweistufigen Reaktion 10 ist der Fluoreszenzfarbstoff 5 in seiner geschützten Form 6 inert gegenüber dem Anregungslicht 16 und dem Fluoreszenzverhinderungslicht 18, so dass während des Abtast- und Detektionsschritts S2 mit Anregungslicht 16 und Fluoreszenzverhinderungslicht 18 beleuchteter, in der geschützten, nicht-fluoreszenten Form 6 vorliegender Fluoreszenzfarbstoff 5 nicht aktiviert wird.

**Fig. 3** zeigt einen von dem aus dem Stand der Technik bekannten *Caged Q-Rhodamin* abgeleiteten Fluoreszenzfarbstoff 5 mit einem sogenannten Carbopyronin-Grundkörper und zwei fotolabilen Schutzgruppen 13 und 14 zur

Verwendung in den erfindungsgemäßen Verfahren. Über die Linkergruppe L kann der Fluoreszenzfarbstoff an eine Struktur in der Probe gebunden werden. Die Schutzgruppen 13, 14 unterbinden die Ausbildung eines fluoreszenten Carbopyronin-Fluorophors an zwei unterschiedlichen Positionen im Molekül 24: Während die o-Nitroveratryloxycarbonylgruppe (NVOC) 13 eine der Aminogruppen blockiert, fixiert die Azidgruppe (Az) 14 die (nicht fluoreszente) Spiroform des Moleküls 24. Der Fluoreszenzfarbstoff 5 liegt folglich so lange in einer geschützten, nicht fluoreszenten Form 6 vor, bis beide Schutzgruppen 13, 14 abgespalten sind. Die Schutzgruppen können mit UV-Licht mit Wellenlängen im Bereich von 320 nm bis 360 nm abgespalten werden, wobei in einem Reaktionsschritt 11 die NVOC-Gruppe 14 unter Decarboxylierung (Abspaltung 12 von $CO_2$) zerfällt, während die Azid-Schutzgruppe 14 in einem weiteren Reaktionsschritt 11 unter Abspaltung 12 eines Stickstoffmoleküls $N_2$ und einer nachgelagerten Wolff-Umlagerung entfernt wird, wodurch der aktivierte, fluoreszente Farbstoff 5, 8 ausgebildet wird.

**Fig. 4** zeigt eine bevorzugte Ausführungsform eines der erfindungsgemäßen Verfahren, bei dem die Abtastpositionen 19 auf den Gitterpunkten 20 eines regelmäßigen, hier kartesischen Gitters 21 angeordnet sind. Wie in der Laserscanningmikroskopie üblich erfolgt die Abtastung der Probe mit Anregungs- und Fluoreszenzverhinderungslicht im Abtast- und Detektionsschritt S2 zeilenweise, und die an jeder Abtastposition 19 detektierte Photonenzahl oder Intensität des Fluoreszenzlichts wird als Helligkeitswert 23 einem der jeweiligen Abtastposition 19 korrespondierendem Bildpixel 22 im Bild 2 zugewiesen. Bei dieser Ausführungsform erfolgen der Abtast- und Detektionsschritt S2 und der Bilderzeugungsschritt S3 nicht notwendigerweise zeitlich getrennt; vielmehr ist es vorteilhaft, das Bild 2 bereits während des Abtastens der Probe zu erzeugen und auf einem Anzeigegerät darzustellen.

Nicht aktivierter Fluoreszenzfarbstoff 5, 6 verhält sich gegenüber dem Anregungs- und Fluoreszenzverhinderungslicht inert und unterliegt somit während des Abtastens der Probe weder einer Fotoaktivierung noch einem Ausbleichen. Indem der Fotoaktivierungsschritt S1, der Abtast-und Detektionsschritt S2 und der Bilderzeugungsschritt S3 wiederholt ausgeführt werden, ermöglicht das erfindungsmäße Verfahren eine mehrfache Bildaufnahme der Struktur 4 auch dann, wenn der fotoaktivierte Fluoreszenzfarbstoff 5, 8 während des Abtastens mit Anregungs-und Fluoreszenzverhinderungslicht ausbleicht und so mit herkömmlichen Bildaufnahmeverfahren eine wiederholte Bildaufnahme nicht möglich wäre. Die bei den Wiederholungen gewonnenen Bilder können einzeln gespeichert werden, beispielsweise um zeitliche Veränderungen in der Probe zu messen, sie können akkumulierend miteinander kombiniert werden, um eine größere Menge der vorhandenen Fluorophore oder weitgehend alle Fluorophore für die Abbildung der Strukturen in der Probe zu nutzen und damit eine optimale kontinuierliche Abbildung der Struktur zu generieren. Es ist auch möglich, aus nacheinander aufgenommenen Einzelbildern relative Verschiebungen oder Driften von Strukturen gegeneinander zu bestimmen, diese zu kompensieren und anschließend akkumulierend eine Abbildung der Struktur zu bestimmen. Es ist weiter möglich, wenn in der Probe oder in Teilbereichen der Probe ein Abbild von Strukturen bereits in ausreichend guter Qualität gewonnen wurde, das Verfahren insgesamt zu beenden oder nur in den Bereichen fortzusetzen, in denen die Bildqualität noch nicht befriedigend ist.

**Fig. 5** zeigt eine weitere bevorzugte Ausführungsform eines erfindungsgemäßen Verfahrens, bei dem nur einzelne, räumlich voneinander getrennte Moleküle 24 des Fluoreszenzfarbstoffs 5 aus der geschützten, nicht fluoreszenten Form 6 in die aktivierte, fluoreszente Form 8 überführt werden. Der (hier nicht dargestellte) Fotoaktivierungsschritt wird dabei so gesteuert, dass die aktivierten Moleküle 8, 24 des Fluoreszenzfarbstoffes 5 einen Abstand 25 voneinander aufweisen, der nicht kleiner ist als ein Mindestabstand *d*. Dieser Mindestabstand *d* bemisst sich dabei nach dem Auflösungsvermögen des zur Vorlokalisation der aktivierten Fluoreszenzfarbstoffmoleküle 5, 8, 24 genutzten Verfahrens (siehe unten) oder alternativ nach dem Maß, mit dem der Ort eines aktivierten Fluoreszenzfarbstoffmoleküls schon allein auf Grund der Kenntnis über den Ort der Aktivierung bekannt ist, und des bei der Abtastung mit Anregungslicht 16 und Fluoreszenzverhinderungslicht 18 erzielten Auflösungsvermögens und darf nicht kleiner als jeder der zugehörigen zwei Werte gewählt werden.

Anders als in der in Fig. 3 dargestellten Verfahrensvariante dienen die in dem Abtast- und Detektionsschritt S2 erhaltenen Photonenzahlen oder Intensitäten von Fluoreszenzlicht hier nicht zur direkten Erzeugung eines Bildes 2 im Sinne einer direkten räumlichen Darstellung der detektierten Helligkeitswerte, sondern zunächst zur präzisen Lokalisation der einzelnen fotoaktivierten Moleküle 8, 24 des Fluoreszenzfarbstoffs 5 in einem Lokalisationsteilschritt S3.1. Zu diesem Zweck werden die Abtastpositionen 19 nicht global auf einem regelmäßigen Gitter angeordnet, sondern in Gruppen von anfangs mindestens drei Abtastpositionen 19 um je ein fotoaktiviertes Fluoreszenzfarbstoffmolekül 5, 8, 24. Hierzu erforderliche Ortsschätzungen 26 der fotoaktivierten Fluoreszenzfarbstoffmoleküle 5, 8, 24 können aus der Aktivierung selbst bekannt sein oder aber mit dem Fachmann bekannten Verfahren der Fluoreszenzmikroskopie gewonnen werden und sind hier nicht näher dargestellt. Im einfachsten Fall kann dies beispielsweise mittels einer Aufnahme eines Epifluoreszenzbilds erfolgen, alternativ kann die Probe auch mit dem Anregungslicht 16 abgetastet werden, um einzelne aktivierte Farbstoffmoleküle 5, 8, 24 in der Probe aufzufinden und eine initiale Ortsschätzung 26 vorzunehmen.

Im nachfolgenden Abtast- und Detektionsschritt S2 werden die aktivierten Farbstoffmoleküle 5, 8, 24 beginnend mit den jeweils mindestens drei Abtastpositionen 19 pro Molekül 24 mit den Intensitätsverteilungen von Anregungslicht und Fluoreszenzverhinderungslicht beleuchtet und an jeder der Abtastpositionen 19 eine Photonenzahl oder Intensität von Fluoreszenzlicht detektiert. Aus der an den jeweils mindestens drei Abtastpositionen 19 detektierten Fluoreszenz

werden nun nach Art einer Triangulation gegenüber den anfänglichen Ortsschätzungen 26 verbesserte Positionsschätzwerte der aktivierten Farbstoffmoleküle 5, 8, 24 berechnet. Aus Gründen der Übersichtlichkeit nicht eingezeichnet ist eine Abtastposition am Ort der anfänglichen Ortsschätzung 26. Eine solche Abtastposition kann genutzt werden, um Mehrdeutigkeiten bei den verbesserten Schätzungen der Orte der aktivierten Farbstoffmoleküle 5, 8, 24 zuverlässig zu vermeiden. Auf Basis dieser verbesserten Ortsschätzungen können nun weitere Abtastpositionen 19 zu jedem aktivierten Farbstoffmolekül 5, 8, 24 festgelegt und das Abtasten mit erhöhter Maximalintensität des Fluoreszenzverhinderungslichts fortgesetzt werden. Dadurch dass die Abtastpositionen 19 zunehmend näher an die tatsächlichen Orte der aktivierten Farbstoffmoleküle 5, 8, 23 heranrücken, befinden sich die Abtastpositionen dann näherungsweise auf Spiralbahnen 28. Der Abtast- und Detektionsschritt S2 wird nach einer vordefinierten Anzahl von Abtastpunkten 18 pro aktiviertem Farbstoffmolekül 5, 8, 24 oder bei Unterschreiten eines maximalen akzeptierten Fehlers der Ortsschätzungen beendet.

Zur Erzeugung des Bildes 2 im Rahmen des Bilderzeugungsschritts S3 werden zunächst in einem Lokalisationsteilschritt S3.1 aus den einander zugeordneten Photonenzahlen oder Intensitäten von Fluoreszenzlicht und den Abtastpositionen 19 finale Koordinaten 29 der aktivierten Farbstoffmoleküle 5, 8, 24 ermittelt. Die Koordinaten 29 der lokalisierten Moleküle 24 werden dann in einem Darstellungsteilschritt S3.2 beispielsweise in Form eines zweidimensionalen Histogramms mit geeigneter Rasterweite dargestellt. Um auf diese Weise ein hochaufgelöstes Abbild der Struktur in der Probe zu erhalten, sind die Verfahrensschritte (inklusive der Fotoaktivierung) zu wiederholen (nicht gezeigt), bis das Histogramm Koordinaten 28 so vieler lokalisierter Farbstoffmoleküle 5, 24 umfasst, dass die mit dem Farbstoff 5 markierte Struktur 4 durchgehend durch lokalisierte Farbstoffmoleküle 5, 24 repräsentiert wird.

In **Fig. 6** sind sechs verschiedene Ausführungsformen A bis F der erfindungsgemäßen Verfahren tabellarisch zusammengestellt. Die Aufstellung ist exemplarisch und stellt keine abschließende Auflistung aller Ausführungsformen der erfindungsgemäßen Verfahren dar.

Den gezeigten Ausführungsformen ist gemeinsam, dass der Fluoreszenzfarbstoff initial in einer geschützten, nicht-fluoreszenten Form vorliegt und dass ein Teil des Fluoreszenzfarbstoffs durch Beleuchten mit Aktivierungslicht in einer mindestens zwei lichtinduzierte Reaktionsschritte umfassenden Reaktion in die aktivierte, fluoreszente Form überführt wird. In der Tabelle sind die die gezeigten Ausführungsformen unterscheidenden Merkmale symbolisch dargestellt. In der zweiten Tabellenspalte ist angegeben, ob in der jeweiligen Ausführungsform eine Fotoaktivierung 9 von einzelnen Molekülen 24 oder von Molekülensembles 30, d.h. mehreren Molekülen innerhalb eines Detektionsvolumens vorgesehen ist. In der dritten Tabellenspalte ist das Abtastschema 36 angegeben, das angibt, ob in der jeweiligen Ausführungsform eine regelmäßige Abtastung 31, insbesondere entlang eines regelmäßigen Gitters 21, oder eine adaptive Abtastung 32 erfolgt, bei der die Abtastpositionen unter Berücksichtigung der in vorhergehenden Abtastschritten detektierten Fluoreszenzsignale festgelegt werden. In der vierten Spalte ist die Intensitätsverteilung des Anregungslichts 16 symbolisch dargestellt, wobei zwischen einer ein zentrales Intensitätsmaximum aufweisenden Intensitätsverteilung 15, einer homogenen Intensitätsverteilung 33 und einer ein zentrales Intensitätsminimum aufweisenden Intensitätsverteilung 17 unterschieden wird. In der fünften Spalte ist dargestellt, ob mit der Intensitätsverteilung des Anregungslichts 16 eine Abtastung 35 erfolgt oder ob die Intensitätsverteilung des Anregungslichts 16 eine ortsfeste Position 34 einnimmt. Die sechste und die siebte Spalten geben die entsprechenden Merkmale für das Fluoreszenzverhinderungslicht 18 wieder.

Die in Zeile A gezeigte, besonders bevorzugte Ausführungsform entspricht der aus der STED-Mikroskopie bekannten Kombination von fokussiertem Anregungslicht 16 mit einer ringförmigen Intensitätsverteilung 17 von Fluoreszenzverhinderungslicht 18 bzw. Stimulationslicht. Beide Intensitätsverteilungen werden gemeinsam und synchron entlang eines regelmäßigen, üblicherweise kartesischen Rasters über die Probe oder einen Ausschnitt der Probe gescannt. Zur Erzeugung eines Rasterbildes wird die an jedem Abtastpunkt detektierte Fluoreszenz einem korrespondierenden Bildpixel als Helligkeitswert zugewiesen. Die in Zeile B gezeigte Variante unterscheidet sich von Ausführungsform A dadurch, dass das Anregungslicht 16 in Form einer im Abtastbereich homogenen Intensitätsverteilung 33 eingestrahlt wird. Die Abtastung 35 erfolgt nur mit der das Intensitätsminimum aufweisenden Intensitätsverteilung 17 von Fluoreszenzverhinderungslicht 18. Aufgrund der Beleuchtung der gesamten Probe (oder zumindest des interessierenden Ausschnitts der Probe) mit Anregungslicht 16 kommt es bei der Ausführungsform B zu vermehrten Beiträgen von Fluoreszenzlicht aus Bereichen außerhalb der aktuellen Abtastposition, weswegen diese Ausführungsform weniger bevorzugt ist. Bei den Ausführungsformen C bis F erfolgt die Fotoaktivierung 9 in Form einzelner, räumlich voneinander getrennter Moleküle 24, so dass beim Abtasten der Probe oder des Ausschnitts der Probe Fluoreszenz von jeweils nur einem aktivierten Farbstoffmolekül detektiert wird. In der besonders bevorzugten Ausführungsform C werden zwei jeweils ein Intensitätsminimum aufweisende Intensitätsverteilungen 17 von Anregungs- und Fluoreszenzverhinderungslicht überlagert, wobei die Probe oder der Ausschnitt der Probe zumindest mit dem Anregungslicht 16 abgetastet wird. Sofern das Fluoreszenzverhinderungslicht 18 beim Abtasten jeweils eines aktivierten Farbstoffmoleküls 24 an einer ortsfesten Position 34 verbleibt, dient das Fluoreszenzverhinderungslicht 18 ganz vorwiegend nur der Unterdrückung von Fluoreszenzbeiträgen aus Bereichen außerhalb der Intensitätsminima; zu diesem Zweck ist eine Intensitätsverteilung mit einem möglichst breiten Intensitätsminimum besonders vorteilhaft. Aus den an den Abtastpositionen detektierten Fluoreszenzsignalen können zuvor ermittelte Positionsschätzungen der aktivierten Farbstoffmoleküle verbessert, d.h. die aktivierten Farbstoffmoleküle zunehmend genauer lokalisiert werden. Die Abtastung erfolgt dabei bevorzugt adaptiv,

d.h. dass die Abtastpunkte unter Berücksichtigung der an vorhergehenden Abtastpositionen detektierten Fluoreszenzsignale festgelegt werden, wobei gleichzeitig die Gesamtintensität des Anregungslichts 16 erhöht wird. Auf diese Weise kann die Lokalisierung der Farbstoffmoleküle mit einer Unsicherheit von wenigen Nanometern erfolgen. In der Ausführungsform C kann das Abtasten optional auch Anregungslicht 16 und Fluoreszenzverhinderungslicht 18 gemeinsam erfolgen; in diesem Fall wird die Fluoreszenzemission an den Abtastpositionen von Anregungs- und Fluoreszenzverhinderungslicht moduliert, was die Berechnung verbesserter Positionsschätzwerte erschweren kann. Die Ausführungsvarianten D und E unterscheiden sich von der Ausführungsvariante C dadurch, dass das Fluoreszenzverhinderungslicht 18 hier nicht nur der Unterdrückung von Fluoreszenzbeiträgen aus Bereichen außerhalb der Intensitätsminima dient, sondern dass das Fluoreszenzverhinderungslicht 18 primär zur Abtastung der Probe oder des Ausschnitts der Probe an den Abtastpositionen eingesetzt wird und die Fluoreszenzemission des abgetasteten Farbstoffmoleküls 24 zur Berechnung verbesserter Positionsschätzwerte moduliert. Das Anregungslicht kann in diesen Ausführungsformen homogen verteilt oder strukturiert sein, zum Beispiel in Form eines gaußförmigen Fokus', und kann ortsfest sein oder zusammen mit dem Fluoreszenzverhinderungslicht 18 beim Abtasten verlagert werden.

Die Ausführungsform F schließlich vereint Merkmale der Ausführungsformen A einerseits sowie C bis E andererseits. Wiederum werden einzelne, räumlich isolierte Farbstoffmoleküle aktiviert, die Abtastung 35 dieser Einzelmoleküle erfolgt jedoch wie in Ausführungsform A, d. h. mit überlagerten Intensitätsverteilungen von Anregungslicht 16 (mit Intensitätsmaximum) und Fluoreszenzverhinderungslicht 18 (mit Intensitätsminimum), wobei die Abtastung 35 entlang eines regelmäßigen Gitters 21 erfolgt. Aufgrund der Fotoaktivierung 9 vereinzelter Farbstoffmoleküle 24 kann aus den an den Abtastpositionen entlang des Gitters 21 detektierten Photonenzahlen oder Intensitäten des Fluoreszenzlichts eine Positionsschätzung der Einzelmoleküle 24 mit einer Unsicherheit erfolgen, die deutlich unterhalb der des Abstands der Abtastpositionen liegen.

**Bezugszeichenliste**

| | |
|---|---|
| S1 | Fotoaktivierungsschritt |
| S1.1 | Vorlokalisationsteilschritt |
| S2 | Abtast- und Detektionsschritt |
| S2.1 | Positionierteilschritt |
| S2.2 | Beleuchtungs- und Detektionsteilschritt |
| S3 | Bilderzeugungsschritt |
| S3.1 | Lokalisationsteilschritt |
| S3.2 | Darstellungsteilschritt |
| 1 | Datenspeicher |
| 2 | Bild |
| 3 | Ausschnitt |
| 4 | Struktur |
| 5 | Fluoreszenzfarbstoff |
| 6 | geschützte, nicht-fluoreszente Form |
| 7 | Aktivierungslicht |
| 8 | aktivierte, fluoreszente Form |
| 9 | Fotoaktivierung |
| 10 | zweistufige Reaktion |
| 11 | Reaktionsschritt |
| 12 | Abspaltung |
| 13 | fotolabile Schutzgruppe |
| 14 | fotolabile Schutzgruppe |
| 15 | Intensitätsverteilung mit lokalem Maximum |
| 16 | Anregungslicht |
| 17 | Intensitätsverteilung mit lokalem Minimum |
| 18 | Fluoreszenzverhinderungslicht |
| 19 | Abtastposition |
| 20 | Gitterpunkt |
| 21 | Gitter |
| 22 | Bildpixel |
| 23 | Helligkeitswert |
| 24 | (Einzel-)Molekül |

| | |
|---|---|
| 25 | Abstand |
| 26 | Positionsschätzwert |
| 28 | Spiralbahn |
| 29 | Koordinaten |
| 30 | Molekülensemble |
| 31 | regelmäßige Abtastung |
| 32 | adaptive Abtastung |
| 33 | homogene Intensitätsverteilung |
| 34 | ortsfeste Position |
| 35 | Abtastung |
| 36 | Abtastschema |

## Patentansprüche

1. Verfahren zur Lokalisation einzelner Moleküle (24) eines Fluoreszenzfarbstoffs (5) in einer Probe umfassend den Verfahrensschritt

    - Auswählen eines Fluoreszenzfarbstoffs (5), der durch Beleuchten mit einem Aktivierungslicht (7) aus einer geschützten, nicht-fluoreszenten Form (6) in eine aktivierte, fluoreszente Form (8) überführbar ist,
    eine erste Gruppe von Verfahrensschritten umfassend die Schritte

        - Fotoaktivieren eines oder mehrerer, räumlich einen Mindestabstand d voneinander entfernter Moleküle (24) des Fluoreszenzfarbstoffs (5) aus der geschützten, nicht-fluoreszenten Form (6) in die aktivierte Form (8) durch Beleuchten mit Aktivierungslicht (7),
        - Bestimmen initialer Positionsschätzwerte (26) eines oder mehrerer aktivierter Farbstoffmoleküle (5, 8, 24) mit einer Unsicherheit von nicht mehr als *d*/2,
        - Ausbilden einer Intensitätsverteilung eines Anregungslichts (16) und einer Intensitätsverteilung eines Fluoreszenzverhinderungslichts (18) in der Probe, wobei zumindest die Intensitätsverteilung des Fluoreszenzverhinderungslichts (18) ein lokales Intensitätsminimum aufweist, wobei auch die Intensitätsverteilung des Anregungslichts (16) ein Intensitätsminimum aufweisen kann;

    und eine zweite Gruppe von Verfahrensschritten umfassend die Schritte

        - Abtasten der Probe oder eines Ausschnitts (3) der Probe mit einer der ein Intensitätsminimum aufweisenden Intensitätsverteilungen (17) an einer Abfolge von Abtastpositionen (19), wobei die Abfolge Untermengen mit jeweils mindestens zwei Abtastpositionen (19) enthält, die in einem Abstand (25) von weniger als d/2 um den Positionsschätzwert (26) eines der Untermenge zugeordneten, aktivierten Farbstoffmoleküls (5, 8, 24) angeordnet sind,
        - Detektieren einer Photonenzahl oder einer Intensität von Fluoreszenzlicht an jeder Abtastposition (19) der Abfolge und Zuordnen der Photonenzahl oder der Intensität zu der jeweiligen Abtastposition (19),
        - Bestimmen eines neuen Positionsschätzwerts (26) für jedes der einer Untermenge zugeordneten aktivierten Farbstoffmoleküle (5, 8, 24) aus den einander zugeordneten Photonenzahlen oder Intensitäten des Fluoreszenzlichts und den Abtastpositionen (19),

    wobei der Wert von d so bemessen ist, dass den aktivierten fluoreszenten Farbstoffmolekülen (5, 8, 24) initiale Positionsschätzwerte (26) eindeutig zugeordnet werden können und dass das detektierte Fluoreszenzlicht an jeder Abtastposition (19) nur von jeweils einem einzigen aktivierten Molekül (8, 24) des Fluoreszenzfarbstoffs (5) stammt,
    **dadurch gekennzeichnet, dass** der Fluoreszenzfarbstoff (5) so ausgewählt wird, dass das Fotoaktivieren mindestens zwei jeweils lichtinduzierte Reaktionsschritte (11) umfasst.

2. Verfahren nach Anspruch 1, **dadurch gekennzeichnet, dass** die Intensitätsverteilung des Anregungslichts (16) ein lokales Intensitätsminimum aufweist und die Probe oder der Ausschnitt (3) der Probe mit der Intensitätsverteilung (17) des Anregungslichts (16) abgetastet wird.

3. Verfahren nach Anspruch 1, **dadurch gekennzeichnet, dass** die Intensitätsverteilung (15) des Anregungslichts (16) ein lokales Intensitätsmaximum aufweist, wobei die Intensitätsverteilungen des Fluoreszenzverhinderungslichts

(18) und des Anregungslichts (16) im Wesentlichen komplementär zueinander sind.

4. Verfahren nach einem der Ansprüche 1 bis 3, **dadurch gekennzeichnet, dass** die zweite Gruppe von Verfahrensschritten wiederholt ausgeführt wird, insbesondere wobei eine Gesamtintensität der ein lokales Intensitätsminimum aufweisenden Intensitätsverteilung (17) des Anregungslichts (16) und / oder des Fluoreszenzverhinderungslichts (18) zwischen den Wiederholungen erhöht und die Abtastpositionen (19) der Untermengen in Richtung des jeweils aktuellen Positionsschätzwertes (26) des zugeordneten aktivierten Fluoreszenzmoleküls (5, 8, 24) verschoben werden.

5. Verfahren nach einem der Ansprüche 1 bis 4, **dadurch gekennzeichnet, dass** eine Bewegung einzelner Moleküle (24) des Fluoreszenzfarbstoffs (5) in einer Probe verfolgt wird, insbesondere wobei die Gesamtintensität der ein lokales Intensitätsminimum aufweisenden Intensitätsverteilung (17) des Anregungslichts (16) und / oder des Fluoreszenzverhinderungslichts (18) zwischen zwei Wiederholungen reduziert und die Abtastpositionen (19) der Untermengen in Richtung eines durch zeitliche Extrapolation ermittelten Positionsschätzwertes (26) des zugeordneten aktivierten Fluoreszenzmoleküls (5, 8, 24) verschoben werden.

6. Verfahren zur Lokalisation einzelner Moleküle (24) eines Fluoreszenzfarbstoffs (5) in einer Probe umfassend den Verfahrensschritt

   - Auswählen eines Fluoreszenzfarbstoffs (5), der durch Beleuchten mit einem Aktivierungslicht (7) aus einer geschützten, nicht-fluoreszenten Form (6) in eine aktivierte, fluoreszente Form (8) überführbar ist,
   sowie eine Gruppe von Verfahrensschritten umfassend die Schritte

     - Fotoaktivieren eines oder mehrerer, räumlich einen Mindestabstand d voneinander entfernter Moleküle des Fluoreszenzfarbstoffs (5) aus der geschützten, nicht-fluoreszenten Form (6) in die aktivierte Form (8) durch Beleuchten mit Aktivierungslicht (7),
     - Ausbilden einer Intensitätsverteilung eines Anregungslichts (16) und einer Intensitätsverteilung eines Fluoreszenzverhinderungslichts (18) in der Probe, wobei die Intensitätsverteilung (17) des Fluoreszenzverhinderungslichts (18) ein lokales Intensitätsminimum aufweist,
     - Abtasten der Probe oder eines Ausschnitts (3) der Probe mit der ein Intensitätsminimum aufweisenden Intensitätsverteilung (17) des Fluoreszenzverhinderungslichts (18) an einer Abfolge von Abtastpositionen (19), die untereinander einen Abstand von nicht mehr als d/2 aufweisen;
     - Detektieren einer Photonenzahl oder einer Intensität von Fluoreszenzlicht an jeder Abtastposition (19) der Abfolge und Zuordnen der Photonenzahl oder der Intensität zu der jeweiligen Abtastposition (19),
     - Lokalisieren von aktivierten Farbstoffmolekülen (5, 8, 24) aus den einander zugeordneten Photonenzahlen oder Intensitäten des Fluoreszenzlichts und den Abtastpositionen (19) mit einer Unsicherheit von höchstens *d*/10 in mindestens einer Raumrichtung,

   wobei der Wert von *d* so bemessen ist, dass das detektierte Fluoreszenzlicht an jeder Abtastposition (19) nur von jeweils einem einzigen aktivierten Molekül (8, 24) des Fluoreszenzfarbstoffs (5) stammt,
   **dadurch gekennzeichnet, dass** der Fluoreszenzfarbstoff (5) so ausgewählt wird, dass das Fotoaktivieren mindestens zwei jeweils lichtinduzierte Reaktionsschritte (11) umfasst.

7. Verfahren nach Anspruch 6, **dadurch gekennzeichnet, dass** die Intensitätsverteilung (15) des Anregungslichts (16) ein lokales Intensitätsmaximum aufweist, wobei die Intensitätsverteilungen des Fluoreszenzverhinderungslichts (18) und des Anregungslichts (16) im Wesentlichen komplementär zueinander sind.

8. Verfahren nach einem der Ansprüche 1 bis 7, **dadurch gekennzeichnet, dass** für das Verfahren gemäß den Ansprüchen 6 oder 7 die Gruppe von Verfahrensschritten gemäß Anspruch 6 wiederholt ausgeführt wird oder für das Verfahren gemäß einem der Ansprüche 1-5 die erste Gruppe und die zweite Gruppe von Verfahrensschritten gemäß Anspruch 1 insgesamt wiederholt ausgeführt werden, wobei zwischen den Wiederholungen die jeweils aktivierten Farbstoffmoleküle (5, 8, 24) in einen nicht-fluoreszenten Zustand überführt werden, insbesondere wobei aus den Lokalisationen der einzelnen Moleküle (24) des Fluoreszenzfarbstoffs (5) oder aus den durch das Lokalisieren bestimmten Orten der aktivierten Farbstoffmoleküle (5, 8, 24) ein räumlich hochaufgelöstes Abbild einer Struktur (4) in der Probe rekonstruiert wird.

9. Verfahren zum Erzeugen räumlich hochaufgelöster Bilder einer Struktur (4) in einer Probe umfassend die Verfahrensschritte

- Auswählen eines Fluoreszenzfarbstoffs (5), der durch Beleuchten mit einem Aktivierungslicht (7) aus einer geschützten, nicht-fluoreszenten Form (6) in eine aktivierte, fluoreszente Form (8) überführbar ist,
- Markieren der Struktur (4) mit dem Fluoreszenzfarbstoff (5),

sowie die einmalig oder wiederholt ausgeführten Verfahrensschritte

- Fotoaktivieren einer Teilmenge des Fluoreszenzfarbstoffs (5) aus der geschützten, nicht-fluoreszenten Form (6) in die aktivierte Form (8) durch Beleuchten mit Aktivierungslicht (7),
- Ausbilden einer Intensitätsverteilung eines Anregungslichts (16) und einer Intensitätsverteilung (17) eines Fluoreszenzverhinderungslichts (18) in der Probe, wobei die Intensitätsverteilung des Fluoreszenzverhinderungslichts (18) ein lokales Intensitätsminimum aufweist,
- Abtasten der Probe oder eines Ausschnitts (3) der Probe mit der ein Intensitätsminimum aufweisenden Intensitätsverteilung (17) des Fluoreszenzverhinderungslichts (18) an einer Abfolge von Abtastpositionen (19),
- Detektieren einer Photonenzahl oder einer Intensität von Fluoreszenzlicht an jeder Abtastposition (19) und Zuordnen der Photonenzahl oder der Intensität zu der jeweiligen Abtastposition (19),
- Erzeugen eines hochaufgelösten Rasterbildes der Struktur (4) aus den einander zugeordneten Photonenzahlen oder Intensitäten des Fluoreszenzlichts und den Abtastpositionen (19), indem jedem Bildpixel (22) des Rasterbildes ein Helligkeitswert (23) zugeordnet wird, der eine monotone Funktion der an der jeweiligen Abtastposition (19) oder einer jeweiligen Menge von Abtastpositionen detektierten Photonenzahl oder Intensität des Fluoreszenzlichts ist,

**dadurch gekennzeichnet, dass** der Fluoreszenzfarbstoff (5) so ausgewählt wird, dass das Fotoaktivieren mindestens zwei jeweils lichtinduzierte Reaktionsschritte (11) umfasst.

**10.** Verfahren nach Anspruch 9, **dadurch gekennzeichnet, dass** mit dem Anregungslicht (16) eine ein lokales Intensitätsmaximum aufweisende, zur Intensitätsverteilung (15) des Fluoreszenzverhinderungslichts (18) im Wesentlichen komplementäre Intensitätsverteilung ausgebildet wird, und dass das Abtasten gemeinsam mit dem Anregungslicht (16) und dem Fluoreszenzverhinderungslicht (18) erfolgt.

**11.** Verfahren nach einem der Ansprüche 1 bis 10, **dadurch gekennzeichnet, dass** mit dem Aktivierungslicht (7) in der Probe eine Mehrzahl von Beleuchtungspunkten ausbildet wird, insbesondere wobei die Beleuchtungspunkte auf einem regelmäßigen Gitter (21) angeordnet werden.

**12.** Verfahren nach einem der Ansprüche 1 bis 11, **dadurch gekennzeichnet, dass** ein lichtinduzierter Reaktionsschritt (11) durch eine Mehrphotonenabsorption induziert wird.

**13.** Verfahren nach einem der Ansprüche 1 bis 12, **dadurch gekennzeichnet, dass** mindestens einer der lichtinduzierten Reaktionsschritte (11) eine fotolytische Abspaltung (12) einer fotolabilen Schutzgruppe (13) ist, insbesondere wobei die fotolabile Schutzgruppe (13) ausgewählt ist aus der Gruppe (jeweils unsubstituiert oder substituiert): Nitrobenzyl, Nitrophenethyl, Nitroindolinyl, Dinitroindolinyl, Nitroveratryl, Arylcarbonylmethyl, Alkylphenacyl, Hydroxyphenacyl, Benzoin, Hydroxycinnamat, o-Nitro-2-phenethyloxy-carbonyl, Nitroanilid, Cumarinyl, Aminocumarinyl, Methoxycumarylmethyl, Anthrachinon-2-ylmethoxycarbonyl, (2-Naphthyl)methyl, (Anthracen-9-yl)methyl, (Pyren-1-yl)methyl, (Perylen-3-yl)methyl, (Phenanthren-9-yl)methyl, o-Hydroxyarylmethyl, Azid, Bordipyrromethenyl.

**14.** Verfahren nach einem der Ansprüche 1 bis 13, **dadurch gekennzeichnet, dass** mindestens zwei der lichtinduzierten Reaktionsschritte (11) Stufen einer Tandem-Reaktion sind, insbesondere wobei eine Stufe der Tandem-Reaktion reversibel ist.

**15.** Verwendung eines Fluoreszenzfarbstoffs (5) in einem Verfahren nach einem der Ansprüche 1 bis 14, **dadurch gekennzeichnet, dass** der Fluoreszenzfarbstoff (5) durch Beleuchten mit einem Aktivierungslicht (7) aus einer geschützten, nicht-fluoreszenten Form (6) in eine aktivierte, fluoreszente Form (8) überführbar ist, und dass das Überführen des Farbstoffs (5) in die fluoreszente Form (8) mindestens zwei jeweils lichtinduzierte Reaktionsschritte (11) umfasst.

## Claims

**1.** A method for localising single molecules (24) of a fluorescent dye (5) in a sample comprising the method step of

- selecting a fluorescent dye (5) that is convertible from a protected, non-fluorescent form (6) into an activated, fluorescent form (8) by illumination with an activation light (7),
a first group of method steps comprising the steps of

- photoactivation of one or more molecules (24) of the fluorescent dye (5), which are spaced at a minimum distance d from each other, from the protected, non-fluorescent form (6) into the activated form (8) by illumination with activation light (7),
- determining initial position estimates (26) of one or more activated dye molecules (5, 8, 24) with an uncertainty of no more than *d*/2,
- forming an intensity distribution of an excitation light (16) and an intensity distribution of a fluorescence inhibition light (18) in the sample, wherein at least the intensity distribution of the fluorescence inhibition light (18) comprises a local intensity minimum, wherein also the intensity distribution of the excitation light (16) may comprise an intensity minimum;

and a second group of method steps comprising the steps of

- scanning the sample or a section (3) of the sample with one of the intensity distributions (17) comprising an intensity minimum at a sequence of scanning positions (19), the sequence containing subsets each comprising at least two scanning positions (19) which are arranged at a distance (25) of less than *d*/2 around the position estimate (26) of an activated dye molecule (5, 8, 24) associated with the subset,
- detecting a photon number or an intensity of fluorescent light at each scanning position (19) of the sequence and associating the photon number or the intensity with the respective scanning position (19),
- determining a new position estimate (26) for each of the activated dye molecules (5, 8, 24) associated with a subset from the associated photon numbers or intensities of the fluorescent light and the scanning positions (19),

wherein the value of d is such that initial position estimates (26) can be unambiguously associated with the activated fluorescent dye molecules (5, 8, 24) and that the detected fluorescent light at each scanning position (19) originates from only a single activated molecule (8, 24) of the fluorescent dye (5) in each case, **characterised in that** the fluorescent dye (5) is selected such that the photoactivation comprises at least two respective light-induced reaction steps (11).

2. The method according to claim 1, **characterised in that** the intensity distribution of the excitation light (16) comprises a local intensity minimum and that the sample or the section (3) of the sample is scanned with the intensity distribution (17) of the excitation light (16).

3. The method according to claim 1, **characterised in that** the intensity distribution (15) of the excitation light (16) comprises a local intensity maximum, wherein the intensity distributions of the fluorescence inhibition light (18) and the excitation light (16) are substantially complementary to each other.

4. The method according to one of the claims 1 to 3, **characterised in that** the second group of method steps is carried out repeatedly, in particular wherein an overall intensity of the intensity distribution (17) of the excitation light (16) and/or the fluorescence inhibition light (18) comprising a local intensity minimum is increased between the repetitions and the scanning positions (19) of the subsets are shifted in the direction of the respective current position estimate (26) of the associated activated fluorescence molecule (5, 8, 24).

5. The method according to one of the claims 1 to 4, **characterised in that** a movement of individual molecules (24) of the fluorescent dye (5) in a sample is tracked, in particular wherein the overall intensity of the intensity distribution (17) of the excitation light (16) and/or the fluorescence inhibition light (18) comprising a local intensity minimum is reduced between two repetitions and the scanning positions (19) of the subsets are shifted in the direction of a position estimate (26) of the associated activated fluorescence molecule (5, 8, 24) which is determined by temporal extrapolation.

6. A method for localising single molecules (24) of a fluorescent dye (5) in a sample comprising the method step of

- selecting a fluorescent dye (5) that is convertible from a protected, non-fluorescent form (6) into an activated, fluorescent form (8) by illumination with an activation light (7),

and a group of method steps comprising the steps of

- photoactivation of one or more molecules of the fluorescent dye (5), which are spaced apart by a minimum distance d from each other, from the protected, non-fluorescent form (6) into the activated form (8) by illumination with activation light (7),
- forming an intensity distribution of an excitation light (16) and an intensity distribution of a fluorescence inhibition light (18) in the sample, wherein the intensity distribution (17) of the fluorescence inhibition light (18) comprises a local intensity minimum,
- scanning the sample or a section (3) of the sample with the intensity distribution (17) of the fluorescence inhibition light (18) comprising an intensity minimum, at a sequence of scanning positions (19) which are spaced apart from one another by a distance of not more than d/2;
- detecting a photon number or an intensity of fluorescent light at each scanning position (19) of the sequence and associating the photon number or the intensity with the respective scanning position (19),
- localising activated dye molecules (5, 8, 24) from the associated photon numbers or intensities of the fluorescent light and the scanning positions (19) with an uncertainty of at most d/10 in at least one spatial direction,

wherein the value of d is such that the detected fluorescent light at each scanning position (19) originates only from a single activated molecule (8, 24) of the fluorescent dye (5),
**characterised in that** the fluorescent dye (5) is selected such that the photoactivation comprises at least two respective light-induced reaction steps (11).

**7.** The method according to claim 6, **characterised in that** the intensity distribution (15) of the excitation light (16) comprises a local intensity maximum, the intensity distributions of the fluorescence inhibition light (18) and the excitation light (16) are essentially complementary to each other.

**8.** The method according to one of the claims 1 to 7, **characterised in that,** for the method according to claims 6 or 7, the group of method steps according to claim 6 is carried out repeatedly or, for the method according to one of the claims 1-5, the first group and the second group of method steps according to claim 1 are carried out repeatedly as a whole, wherein between the repetitions the respective activated dye molecules (5, 8, 24) are converted into a non-fluorescent state, in particular wherein a spatially high-resolution image of a structure (4) in the sample is reconstructed from the localisations of the individual molecules (24) of the fluorescent dye (5) or from the locations of the activated dye molecules (5, 8, 24) determined by the localisation.

**9.** A method for generating spatially high-resolution images of a structure (4) in a sample comprising the method steps of

- selecting a fluorescent dye (5) that is convertible from a protected, non-fluorescent form (6) into an activated, fluorescent form (8) by illumination with an activation light (7),
- labelling the structure (4) with the fluorescent dye (5),

as well as the method steps carried out once or repeatedly

- photoactivation of a subset of the fluorescent dye (5) from the protected, non-fluorescent form (6) to the activated form (8) by illumination with activation light (7),
- forming an intensity distribution of an excitation light (16) and an intensity distribution (17) of a fluorescence inhibition light (18) in the sample, wherein the intensity distribution of the fluorescence inhibition light (18) comprises a local intensity minimum,
- scanning the sample or a section (3) of the sample with the intensity distribution (17) of the fluorescence inhibition light (18) comprising an intensity minimum at a sequence of scanning positions (19),
- detecting a photon number or an intensity of fluorescent light at each scanning position (19) and associating the photon number or the intensity with the respective scanning position (19),
- generating a high-resolution raster image of the structure (4) from the associated photon numbers or intensities of the fluorescent light and the scanning positions (19) by associating with each image pixel (22) of the raster image a brightness value (23) that is a monotonic function of the photon number or intensity of the fluorescent light detected at the respective scanning position (19) or a respective set of scanning positions,

**characterised in that** the fluorescent dye (5) is selected such that the photoactivation comprises at least two respective light-induced reaction steps (11).

**10.** The method according to claim 9, **characterised in that** an intensity distribution which comprises a local intensity maximum and is substantially complementary to the intensity distribution (15) of the fluorescence inhibition light (18), is formed by the excitation light and that the scanning is performed together with the excitation light (16) and the fluorescence inhibition light (18).

**11.** The method according to one of the claims 1 to 10, **characterised in that** the activation light (7) is used to form a plurality of illumination points in the sample, in particular wherein the illumination points are arranged on a regular grid (21).

**12.** The method according to one of the claims 1 to 11, **characterised in that** a light-induced reaction step (11) is induced by multiphoton absorption.

**13.** The method according to one of the claims 1 to 12, **characterised in that** at least one of the light-induced reaction steps (11) is a photolytic cleavage (12) of a photolabile protecting group (13), in particular wherein the photolabile protecting group (13) is selected from the group (in each case unsubstituted or substituted): nitrobenzyl, nitrophenethyl, nitroindolinyl, dinitroindolinyl, nitroveratryl, arylcarbonylmethyl, alkylphenacyl, hydroxyphenacyl, benzoin, hydroxycinnamate, o-nitro-2-phenethyloxy carbonyl, nitroanilide, coumarinyl, aminocoumarinyl, methoxycoumarylmethyl, anthraquinon-2-ylmethoxycarbonyl, (2-naphthyl)methyl, (anthracen-9-yl)methyl, (pyren-1-yl)methyl, (perylen-3-yl)methyl, (phenanthren-9-yl)methyl, o-hydroxyarylmethyl, azide, bordipyrro methenyl.

**14.** The method according to one of the claims 1 to 13, **characterised in that** at least two of the light-induced reaction steps (11) are steps of a tandem reaction, in particular wherein one step of the tandem reaction is reversible.

**15.** A use of a fluorescent dye (5) in a method according to one of the claims 1 to 14, **characterised in that** the fluorescent dye (5) is convertible from a protected, non-fluorescent form (6) into an activated, fluorescent form (8) by illumination with an activation light (7), and **in that** the conversion of the dye (5) into the fluorescent form (8) comprises at least two respective light-induced reaction steps (11).

## Revendications

**1.** Procédé de localisation de molécules individuelles (24) d’un colorant fluorescent (5) dans un échantillon comprenant l’étape de procédé suivante

- sélectionner un colorant fluorescent (5) qui peut être converti d’une forme non fluorescente protégée (6) en une forme fluorescente activée (8) par illumination avec une lumière d’activation (7),
un premier groupe d’étapes de procédé comprenant les étapes suivantes

  - photoactivation d’une ou de plusieurs molécules du colorant fluorescent (5) espacées d’une distance minimale *d* de la forme non fluorescente protégée (6) dans la forme activée (8) par illumination avec une lumière d’activation (7),
  - déterminer des estimations initiales de position (26) d’une ou de plusieurs molécules de colorant activées (5, 8, 24) avec une incertitude non supérieure à *d*/2,
  - former une distribution d’intensité d’une lumière d’excitation (16) et une distribution d’intensité d’une lumière d’inhibition de fluorescence (18) dans l’échantillon, au moins la distribution d’intensité de la lumière d’inhibition de fluorescence (18) ayant un minimum d’intensité local, dans lequel la distribution d’intensité de la lumière d’excitation (16) peut également avoir un minimum d’intensité ;

et un deuxième groupe d’étapes de procédé comprenant les étapes de

  - balayage de l’échantillon ou d’une partie (3) de l’échantillon avec l’une des distributions d’intensité (17) présentant un minimum d’intensité à une succession de positions de balayage (19), la succession comprenant des sous-ensembles ayant chacun au moins deux positions de balayage (19), qui sont espacées d’une distance (25) de moins de d/2 autour de l’estimation de position (26) d’une molécule de colorant activée (5, 8, 24) associée au sous-ensemble,
  - détecter un nombre de photons ou une intensité de lumière fluorescente à chaque position de balayage (19) de la succession et associer le nombre de photons ou l’intensité à la position de balayage (19) respective,
  - déterminer une nouvelle estimation de position (26) pour chacune des molécules de colorant activées (5,

8, 24) associées à un sous-ensemble à partir des nombres de photons ou des intensités de la lumière de fluorescence associés les uns aux autres et des positions de balayage (19),

la valeur de d est telle que des estimations de position initiales (26) peuvent être attribuées sans ambiguïté aux molécules de colorant fluorescent activées (5, 8, 24) et que la lumière fluorescente détectée à chaque position de balayage (19) ne provient que d'une seule molécule activée (8, 24) du colorant fluorescent (5),
**caractérisé en ce que** le colorant fluorescent (5) est choisi de telle sorte que la photoactivation comprenne au moins deux étapes de réaction (11) chacune induite par la lumière.

2. Procédé selon la revendication 1, **caractérisé en ce que** la distribution d'intensité de la lumière d'excitation (16) présente un minimum local d'intensité et l'échantillon ou la section (3) de l'échantillon est balayé(e) avec la distribution d'intensité (17) de la lumière d'excitation (16).

3. Procédé selon la revendication 1, **caractérisé en ce que** la distribution d'intensité (15) de la lumière d'excitation (16) présente un maximum local d'intensité, les distributions d'intensité de la lumière d'inhibition de fluorescence (18) et de la lumière d'excitation (16) étant sensiblement complémentaires l'une de l'autre.

4. Procédé selon l'une des revendications 1 à 3, **caractérisé en ce que** le deuxième groupe d'étapes de procédé est exécuté de manière répétée, en particulier dans lequel une intensité totale de la distribution d'intensité (17) de la lumière d'excitation (16) et/ou de la lumière d'inhibition de fluorescence (18) présentant un minimum local d'intensité est augmentée entre les répétitions et les positions de balayage (19) des sous-ensembles sont déplacées en direction de la valeur estimée de position actuelle respective (26) de la molécule fluorescente activée associée (5, 8, 24).

5. Procédé selon l'une des revendications 1 à 4, **caractérisé en ce qu'**un mouvement de molécules individuelles (24) du colorant fluorescent (5) est suivi dans un échantillon, en particulier dans lequel l'intensité totale de la distribution d'intensité (17) de la lumière d'excitation (16) et/ou de la lumière d'inhibition de fluorescence (18), présentant un minimum local d'intensité, est réduite entre deux répétitions et les positions de balayage (19) des sous-ensembles sont déplacées en direction d'une valeur estimée de position (26), déterminée par extrapolation temporelle, de la molécule fluorescente activée associée (5, 8, 24).

6. Procédé de localisation de molécules individuelles (24) d'un colorant fluorescent (5) dans un échantillon comprenant l'étape de procédé suivante

- sélectionner un colorant fluorescent (5) qui peut être converti d'une forme non fluorescente protégée (6) en une forme fluorescente activée (8) par illumination avec une lumière d'activation (7),
ainsi qu'un groupe d'étapes de procédé comprenant les étapes suivantes

    - photoactivation d'une ou de plusieurs molécules du colorant fluorescent (5) espacées d'une distance minimale d de la forme non fluorescente protégée (6) dans la forme activée (8) par illumination avec une lumière d'activation (7),
    - former une distribution d'intensité d'une lumière d'excitation (16) et une distribution d'intensité d'une lumière d'inhibition de fluorescence (18) dans l'échantillon, dans lequel la distribution d'intensité (17) de la lumière d'inhibition de fluorescence (18) présente un minimum local d'intensité,
    - balayage de l'échantillon ou d'une partie (3) de l'échantillon avec la distribution d'intensité (17) de la lumière d'inhibition de fluorescence (18) présentant un minimum d'intensité, à une succession de positions de balayage (19) qui sont espacées d'une distance non supérieure à d/2;
    - détecter un nombre de photons ou une intensité de lumière fluorescente à chaque position de balayage (19) de la succession et associer le nombre de photons ou l'intensité à la position de balayage (19) respective,
    - localisation de molécules de colorant activées (5, 8, 24) à partir des nombres de photons ou des intensités de la lumière de fluorescence associés les uns aux autres et des positions de balayage (19) avec une incertitude d'au plus d/10 dans au moins une direction spatiale,

la valeur de d est telle que la lumière fluorescente détectée à chaque position de balayage (19) ne provient que d'une seule molécule activée (8, 24) du colorant fluorescent (5),
**caractérisé en ce que** le colorant fluorescent (5) est choisi de telle sorte que la photoactivation comprenne au moins deux étapes de réaction (11) chacune induite par la lumière.

7. Procédé selon la revendication 6, **caractérisé en ce que** la distribution d'intensité (15) de la lumière d'excitation (16) présente un maximum local d'intensité, les distributions d'intensité de la lumière d'inhibition de fluorescence (18) et de la lumière d'excitation (16) étant sensiblement complémentaires l'une de l'autre.

8. Procédé selon l'une des revendications 1 à 7, **caractérisé en ce que,** pour le procédé selon les revendications 6 ou 7, le groupe d'étapes de procédé selon la revendication 6 est exécuté de manière répétée ou, pour le procédé selon l'une des revendications 1 à 5, le premier groupe et le deuxième groupe d'étapes de procédé selon la revendication 1 sont exécutés de manière répétée dans leur ensemble, dans lequel, entre les répétitions, les molécules de colorant (5, 8, 24) respectivement activées sont transformées en un état non fluorescent, en particulier dans lequel une image à haute résolution spatiale d'une structure (4) dans l'échantillon est reconstruite à partir des localisations des molécules individuelles (24) du colorant fluorescent (5) ou à partir des emplacements des molécules de colorant activées (5, 8, 24) déterminés par la localisation.

9. Procédé de production d'images à haute résolution spatiale d'une structure (4) dans un échantillon, comprenant les étapes de procédé suivantes

   - sélectionner un colorant fluorescent (5) qui peut être converti d'une forme non fluorescente protégée (6) en une forme fluorescente activée (8) par illumination avec une lumière d'activation (7),
   - marquer la structure (4) avec le colorant fluorescent (5),

   ainsi que les étapes de procédé exécutées une seule fois ou de manière répétée

   - photoactivation d'une quantité partielle du colorant fluorescent (5) de la forme non fluorescente protégée (6) dans la forme activée (8) par illumination avec une lumière d'activation (7),
   - former une distribution d'intensité d'une lumière d'excitation (16) et une distribution d'intensité (17) d'une lumière d'inhibition de fluorescence (18) dans l'échantillon, la distribution d'intensité de la lumière d'inhibition de fluorescence (18) ayant un minimum d'intensité local,
   - balayage de l'échantillon ou d'une partie (3) de l'échantillon avec la distribution d'intensité (17) de la lumière d'inhibition de fluorescence (18) présentant un minimum d'intensité à une succession de positions de balayage (19),
   - détecter un nombre de photons ou une intensité de lumière fluorescente à chaque position de balayage (19) et associer le nombre de photons ou l'intensité à la position de balayage (19) respective,
   - génération d'une image tramée à haute résolution de la structure (4) à partir des nombres de photons ou des intensités de la lumière fluorescente associés les uns aux autres et des positions de balayage (19), en associant à chaque pixel d'image (22) de l'image tramée une valeur de luminosité (23) qui est une fonction monotone du nombre de photons ou de l'intensité de la lumière fluorescente détectée à la position de balayage (19) respective ou à un ensemble respectif de positions de balayage,

   **caractérisé en ce que** le colorant fluorescent (5) est choisi de telle sorte que la photoactivation comprenne au moins deux étapes de réaction (11) chacune induite par la lumière.

10. Procédé selon la revendication 9, **caractérisé en ce que** l'on forme avec la lumière d'excitation (16) une distribution d'intensité présentant un maximum local d'intensité et sensiblement complémentaire de la distribution d'intensité (15) de la lumière d'inhibition de fluorescence (18), et **en ce que** le balayage est effectué conjointement avec la lumière d'excitation (16) et la lumière d'inhibition de fluorescence (18).

11. Procédé selon l'une des revendications 1 à 10, **caractérisé en ce que** l'on forme une pluralité de points d'illumination dans l'échantillon avec la lumière d'activation (7), en particulier dans lequel les points d'illumination sont disposés sur un réseau régulier (21).

12. Procédé selon l'une des revendications 1 à 11, **caractérisé en ce qu'**une étape de réaction induite par la lumière (11) est induite par une absorption multiphotonique.

13. Procédé selon l'une des revendications 1 à 12, **caractérisé en ce qu'**au moins l'une des étapes de réaction (11) induites par la lumière est une élimination photolytique (12) d'un groupe protecteur photolabile (13), en particulier dans lequel le groupe protecteur photolabile (13) est choisi dans le groupe (respectivement non substitué ou substitué) : nitrobenzyle, nitrophénéthyle, nitroindolinyle, dinitroindolinyle, nitroveratryle, arylcarbonylméthyle, alkylphénacyle, hydroxyphénacyle, benzoïne, hydroxycinnamate, o-nitro-2-phénéthyloxy carbonyle, nitroanilide, coumarinyle, aminocoumarinyle, méthoxycumarylméthyle, anthraquinone-2-ylméthoxycarbonyle, (2-naphtyl)méthyle, (anthracène-9-yl)méthyle, (pyrène-1-yl)méthyle, (pérylène-3-yl)méthyle, (phénanthrène-9-yl)méthyle, o-hydroxyarylméthyle, azide, bordipyrro méthényle.

**14.** Procédé selon l'une des revendications 1 à 13, **caractérisé en ce qu'**au moins deux des étapes de réaction (11) induites par la lumière sont des étapes d'une réaction en tandem, en particulier dans lequel une étape de la réaction en tandem est réversible.

**15.** Utilisation d'un colorant fluorescent (5) dans un procédé selon l'une des revendications 1 à 14, **caractérisée en ce que** le colorant fluorescent (5) peut être transféré d'une forme non fluorescente protégée (6) à une forme fluorescente activée (8) par illumination avec une lumière d'activation (7), et **en ce que** le transfert du colorant (5) à la forme fluorescente (8) comprend au moins deux étapes de réaction (11) induites chacune par la lumière.

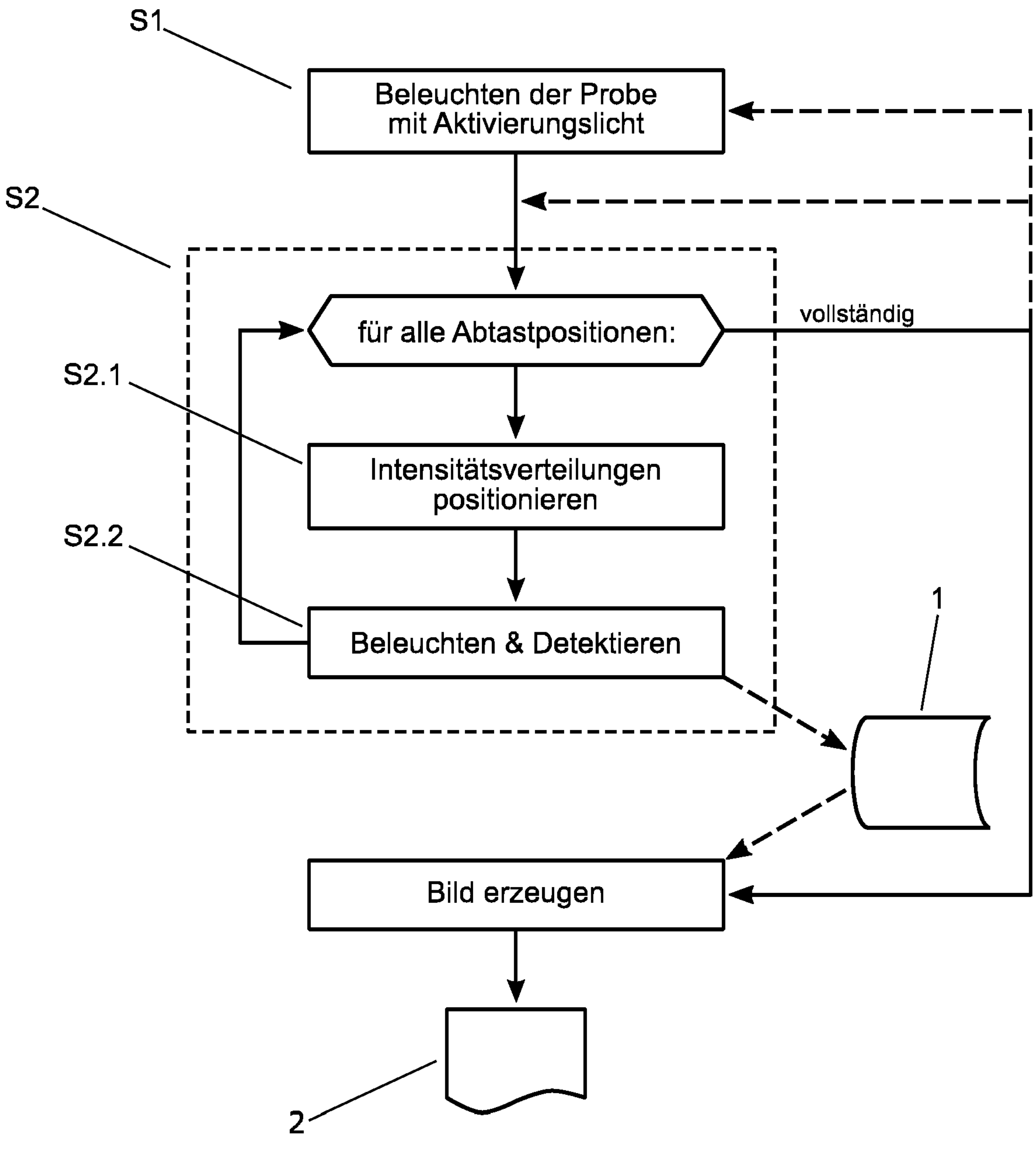
S1
Beleuchten der Probe mit Aktivierungslicht
S2
für alle Abtastpositionen:
vollständig
S2.1
Intensitätsverteilungen positionieren
S2.2
Beleuchten & Detektieren
1
Bild erzeugen
2

Fig. 1

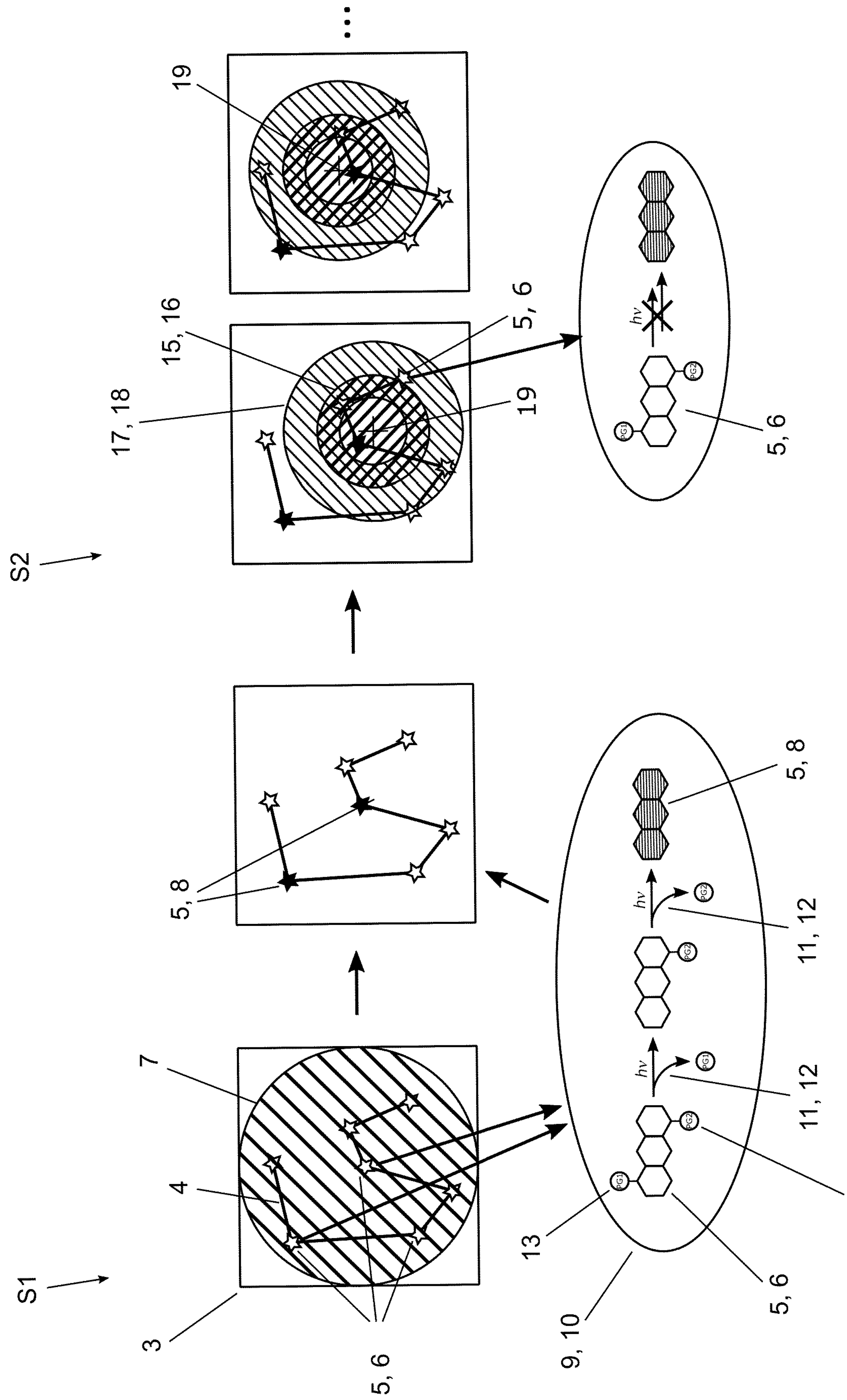


Fig. 2

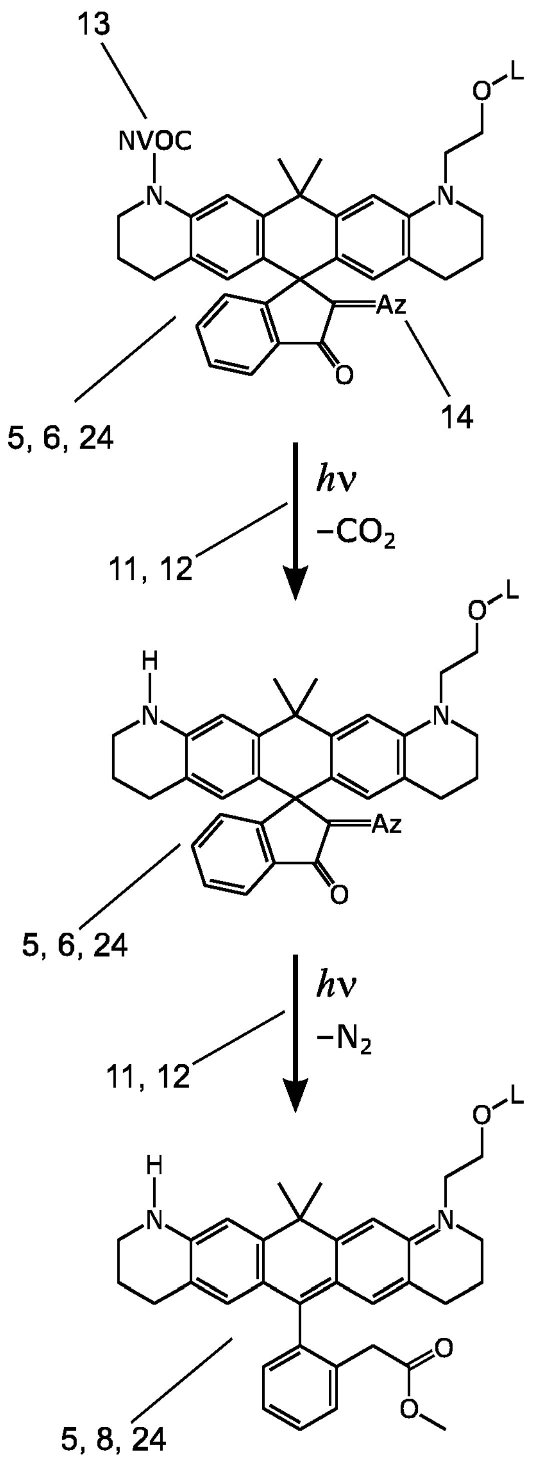


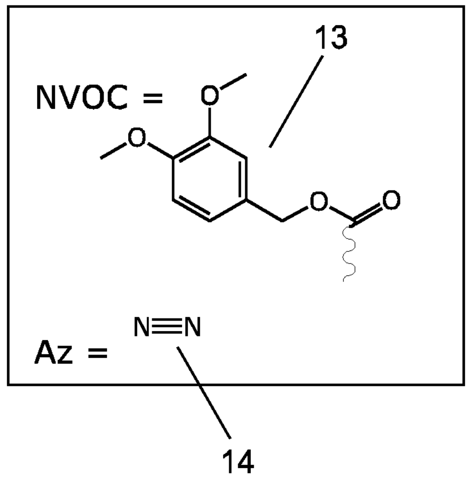


Fig. 3

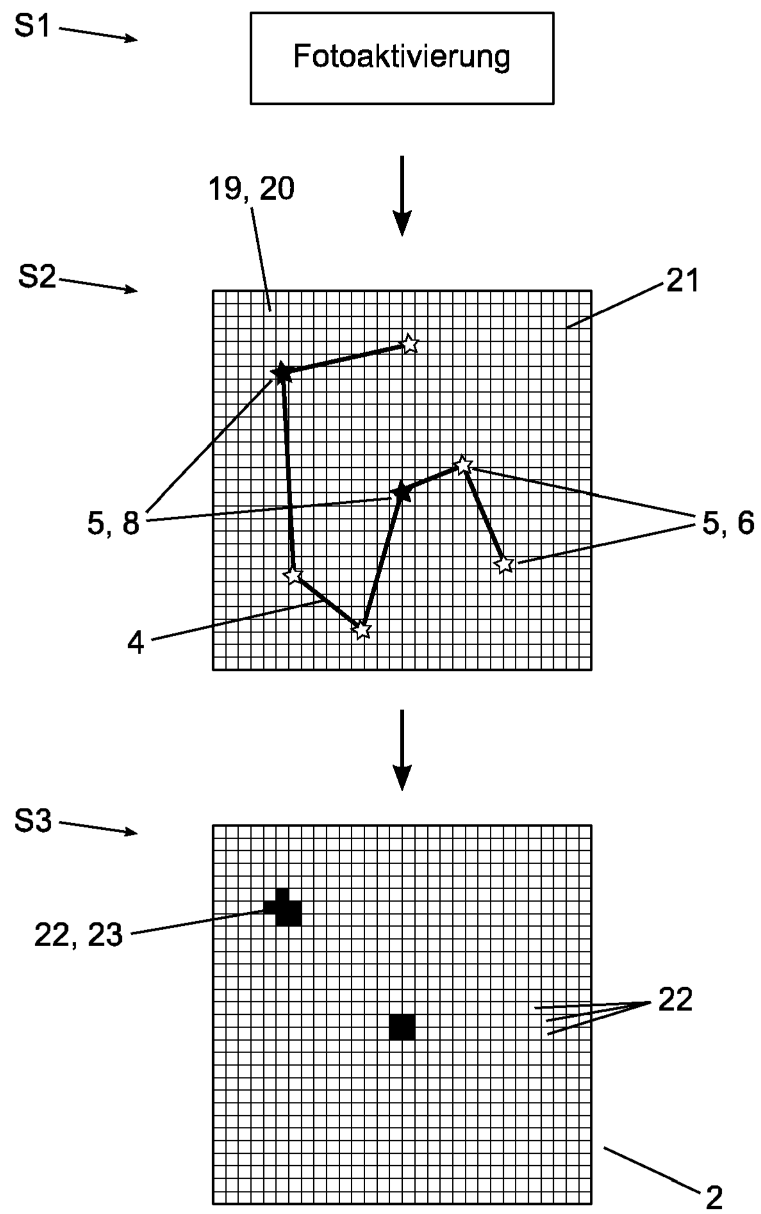


Fig. 4

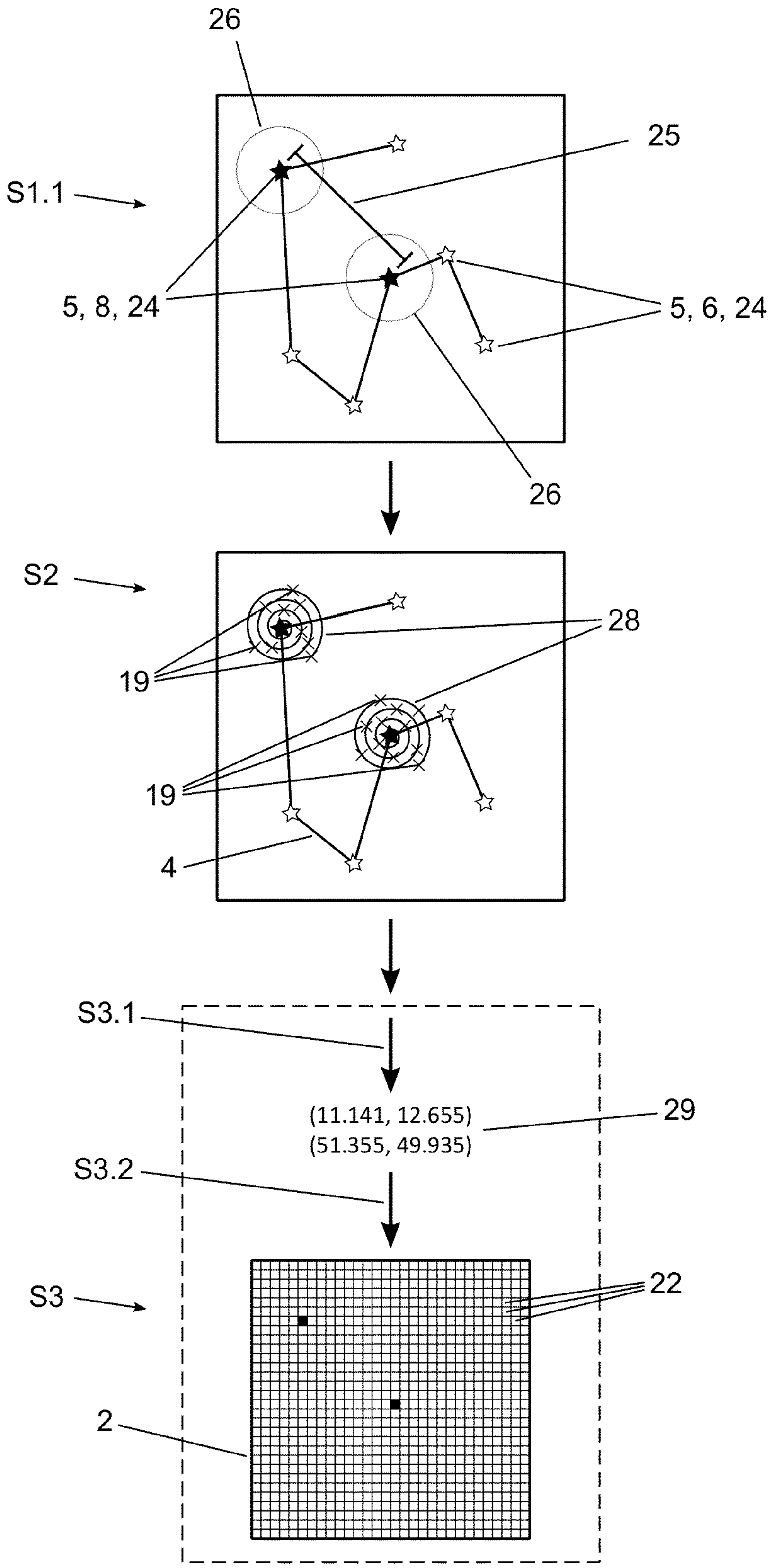
26
25
S1.1
5, 8, 24
5, 6, 24
26
S2
28
19
19
4
S3.1
(11.141, 12.655)
(51.355, 49.935)
29
S3.2
S3
22
2

Fig. 5

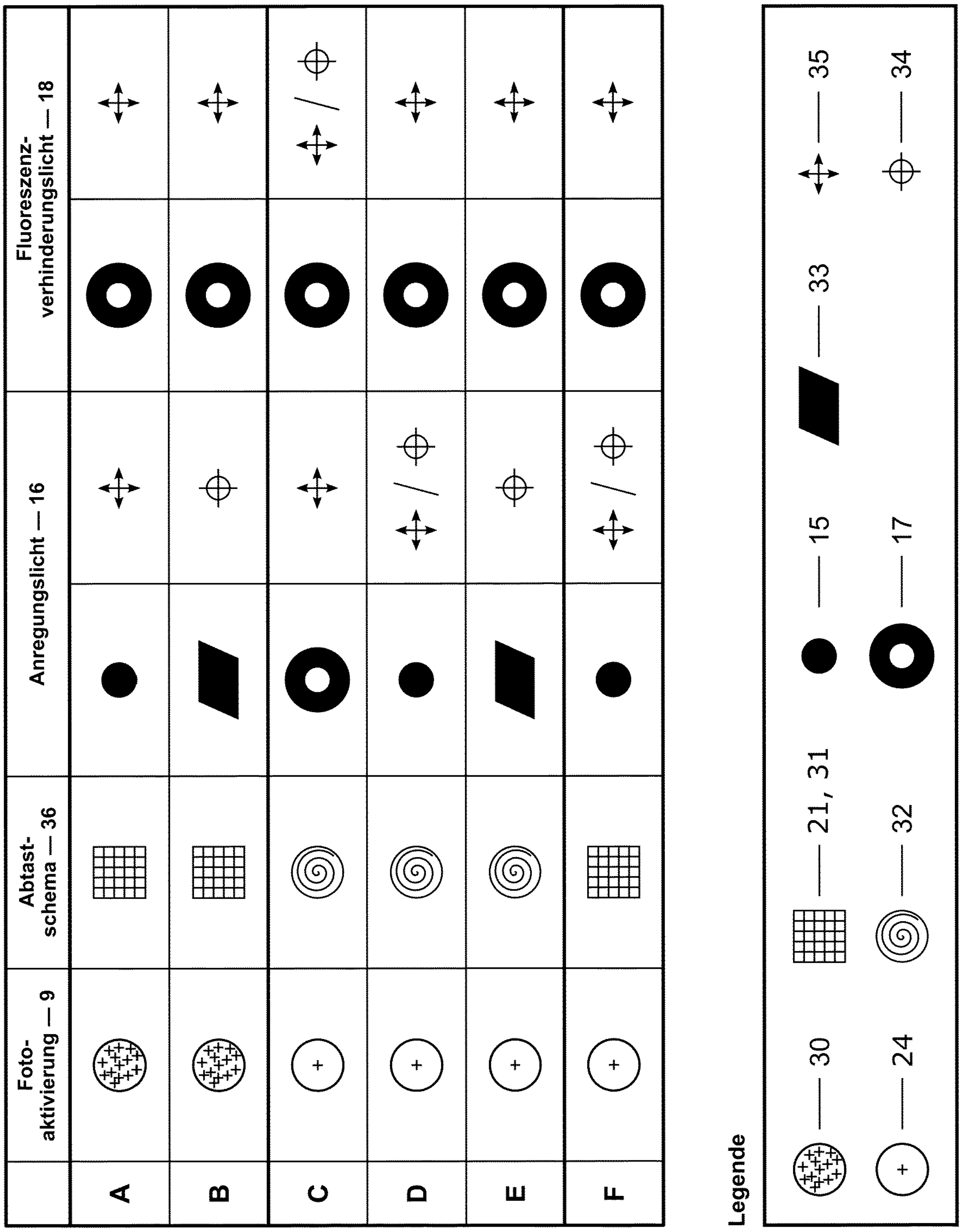

Foto-aktivierung — 9
Abtast-schema — 36
Anregungslicht — 16
Fluoreszenz-verhinderungslicht — 18
A
B
C
D
E
F
Legende
30
21, 31
15
33
35
24
32
17
34


Fig. 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102013100172 A1
- WO 2015097000 A1
- DE 102017104736 B3


### In der Beschreibung aufgeführte Nicht-Patentliteratur


- **R.E. THOMPSON et al.** *Biophys. J.,* 2002, vol. 82, 2775
- **F. BALZAROTTI et al.** *arXiv:1611.03401*
- **VON S.T. HESS ; T.P.K. GIRIRAJAN ; M.D. MASON.** Ultra-High Resolution Imaging by Fluorescence Photoactivation Localization Microscopy. *Biophysical Journal,* 2006, vol. 91, 4258-4272
- **Y. CHEN ; P.J. MACDONALD ; J.P. SKINNER ; G.H. PATTERSON ; J.D. MULLER.** Probing nucleocytoplasmic transport by two-photon activation of PA-GFP. *Microsc.Res.Tech.,* 2006, vol. 69, 220-226
- **VON P. KLÄN et al.** Photoremovable Protecting Groups in Chemistry and Biology: Reaction Mechanisms and Efficacy. *Chem. Rev.,* 2013, vol. 113, 119
- **S. HAUKE et al.** Specific protein labeling with caged fluorophores for dual-color imaging and super-resolution microscopy in living cells. *Chem. Sci.,* 2017, vol. 8, 559
- **P. P. GOSWAMI et al.** BODIPY-Derived Photoremovable Protecting Groups Unmasked with Green Light. *J. Am. Chem. Soc.,* 2015, vol. 137, 3783
- **M.J. HANSEN et al.** Wavelength-Selective Cleavage of Photoprotecting Groups: Strategies and Applications in Dynamic Systems. *Chem. Soc. Rev.,* 2015, vol. 44, 3358
- **VON A. PAUL et al.** o-Hydroxycinnamate for Sequential Photouncaging of Two Different Functional Groups and its Application in Releasing Cosmeceuticals. *Org. Biomol. Chem.,* 2019, vol. 33
- **A. CHMYROV et al.** Nanoscopy with more than 100,000 'doughnuts. *Nature Meth.,* 2013, vol. 10, 737